# EUROPEAN PATENT APPLICATION

(11) **EP 4 840 959 A1**
(43) Date of publication of application: **18.11.2026**
(21) Application number: 26178876.4
(22) Date of filing: 15.05.2026
(51) Int. Cl.: C12N 15/113

(54) **A NUCLEIC ACID MOLECULE FOR SILENCING THE EXPRESSION OF THE GENE ENCODING THE TMPRSS-2 PROTEIN, EXPRESSION CASSETTE, EXPRESSION VECTOR, PHARMACEUTICAL COMPOSITION, NUCLEIC ACID MOLECULE FOR THERAPEUTIC USE, NUCLEIC ACID MOLECULE FOR USE IN THE TREATMENT AND/OR PREVENTION OF A TMPRSS-2-DEPENDENT VIRAL DISEASE, AND AN IN VITRO METHOD FOR SILENCING 5**

(30) Priority: 16.05.2025 PL 45205925
(71) Applicant: Instytut Chemii Bioorganicznej Polskiej Akademii Nauk, 61-704 Poznan (PL)
(72) Inventor: Belter, Agnieszka, 62-023 Kamionki (PL); Olejniczak, Marta, 61-306 Poznan (PL); Synak, Jaroslaw, 61-131 Poznan (PL); Szachniuk, Marta, 61-578 Poznan (PL)
(74) Representative: Pietruszynska, Elzbieta

(57) **Abstract**

The invention relates to a nucleic acid molecule for silencing the expression of the gene encoding the transmembrane serine protease type 2 (TMPRSS-2) protein in a cell, an expression cassette for silencing the expression of the gene encoding the TMPRSS-2 protein containing such a nucleic acid molecule, an expression vector for silencing the expression of the gene encoding the TMPRSS-2 protein comprising such a nucleic acid molecule or such an expression cassette, a pharmaceutical composition for silencing the expression of the gene encoding the TMPRSS-2 protein in an organism comprising such a nucleic acid molecule or such an expression cassette or such an expression vector and a pharmaceutically acceptable carrier. The invention also relates to such a nucleic acid molecule for use in therapy, for use in the treatment and/or prevention of a viral disease dependent on a type 2 transmembrane serine protease, preferably in the treatment and/or prevention of severe acute respiratory syndrome (SARS), Middle East Respiratory Syndrome (MERS), Coronavirus Disease 2019 (COVID-19), influenza, parainfluenza, respiratory syncytial virus (RSV) infection, and Zika fever. The present invention further relates to an *in vitro* method for silencing the expression of the gene encoding transmembrane serine protease 2 (TMPRSS-2) in a cell, wherein such a nucleic acid molecule or cassette or vector is introduced into the cell and the cell is maintained with the introduced nucleic acid molecule or expression cassette or vector under conditions and for a duration sufficient to achieve silencing of the gene encoding the TMPRSS-2 in the cell.

## Description

### FIELD OF THE INVENTION

The subject matter of the present invention is a nucleic acid molecule for silencing the expression of the gene encoding the transmembrane serine protease type 2 (TMPRSS-2) in a cell, an expression cassette for silencing the expression of the gene encoding the TMPRSS-2 protein comprising such a nucleic acid molecule, an expression vector for silencing the expression of the gene encoding the TMPRSS-2 gene containing such a nucleic acid molecule or such an expression cassette, a pharmaceutical composition for silencing the expression of the gene encoding the TMPRSS-2 protein in an organism containing such a nucleic acid molecule or such an expression cassette or such an expression vector and a pharmaceutically acceptable carrier. The invention also relates to such a nucleic acid molecule for use in therapy, for use in the treatment and/or prevention of a TMPRSS-2-dependent viral disease, preferably in the treatment and/or prevention of severe acute respiratory syndrome (SARS), Middle East Respiratory Syndrome (MERS), Coronavirus Disease 2019 (COVID-19), influenza, parainfluenza, respiratory syncytial virus (RSV) infection, and Zika fever by silencing the expression of the gene encoding the TMPRSS-2 protein. The present invention further relates to an *in vitro* method for silencing the expression of the gene encoding TMPRSS-2 in a cell, in which such a nucleic acid molecule or cassette or vector is introduced into the cell and the cell is maintained with the introduced nucleic acid molecule or expression cassette or vector under conditions and for a duration sufficient to achieve silencing of the gene encoding the TMPRSS-2 protein-2 in the cell.

The invention relates to the field of gene expression silencing using RNA interference (RNAi) technology reagents, in particular artificial microRNAs (artificial miRNAs), otherwise referred to in this patent specification as artificial miRNAs or amiRNAs.

### BACKGROUND OF THE INVENTION

TMPRSS-2 (transmembrane serine protease 2) is a serine protease responsible for the proteolytic activation of many viral proteins, including those of viruses causing severe acute respiratory syndrome (SARS), Middle East Respiratory Syndrome (MERS), Coronavirus Disease 2019 (COVID-19), influenza, and Zika fever. It has been demonstrated that critically ill COVID-19 patients had higher levels of TMPRSS-2, and these levels were associated with lower chances of survival. TMPRSS-2 levels can be used to predict COVID-19 mortality in hospitalized patients (Mohan et al., 2024).

Previous studies have shown that inhibiting the expression or activity of TMPRSS-2 reduces viral entry into host cells. Furthermore, knocking out the TMPRSS-2 gene does not induce a phenotype (T. S. Kim et al., 2006), suggesting that this protease can be used as a therapeutic target without causing serious adverse side effects.

### STATE OF THE ART

### Targeting TMPRSS-2 as an Antiviral Therapy

To date, antisense oligonucleotides (ASOs) have been used to reduce TMPRSS-2 expression, which reduced influenza A virus (IAV) infection and SARS-CoV-2 viral entry in cellular models (Nowak et al., 2024) .

Two well-known TMPRSS-2 inhibitors, camostat and nafamostat-approved drugs for the treatment of pancreatitis-are currently undergoing clinical trials as potential treatments for COVID-19 (Mantzourani, Vasilakaki, et al., 2022). These compounds and their derivatives also act as inhibitors of serine proteases such as trypsin, prostasin, matrypsin, plasmin, kallikrein, thrombin, and C1 esterase-enzymes involved in maintaining normal homeostasis, particularly digestion, blood circulation, and coagulation (Nimishakavi et al., 2015). When administered intravenously, they have a short plasma half-life (23 min for nafamostat) (Mantzourani et al., 2022); a clinical evaluation of intravenous (IV) camostat and nafamostat in the treatment of COVID-19 showed moderate or no significant benefits for patients (Gunst et al., 2021; Y. Kim et al., 2023). Treatment of COVID-19 patients with aprotinin, a protease inhibitor active against TMPRSS-2 and other proteases, proved safe and effective in treating COVID-19 (Redondo-Calvo et al., 2022).

To the best of our knowledge, there are no reports on the use of artificial microRNAs (artificial miRNAs), also referred to in this patent description as artificial miRNAs or amiRNAs, to silence the expression of the TMPRSS-2 gene.

### Currently used antiviral drugs and vaccines:

Currently used next-generation antiviral drugs include neuraminidase inhibitors, namely oseltamivir and zanamivir (effective against both influenza A and B viruses).

Currently used vaccines are seasonal vaccines containing split, inactivated influenza viruses (e.g., Fluarix Tetra, Vaxigrip Tetra) or inactivated influenza virus surface antigens: hemagglutinin and neuraminidase (e.g., Influvac Tetra). All influenza vaccines approved for marketing in Poland are produced using influenza viruses grown in chicken embryos.

### Artificial miRNAs (amiRNAs) in the treatment and prevention of viral diseases:

AmiRNAs have been used to combat viral diseases by directly targeting viral RNA, including that of the Chikungunya virus, HIV-1, hepatitis B, influenza, dengue virus, West Nile virus, and Japanese encephalitis virus (Gao et al., 2008; Karothia et al., 2020; Kotowska-Zimmer et al., 2021; Sharma et al., 2018; Xie et al., 2013; Zhang et al., 2012). One limitation is that viruses can mutate very rapidly; therefore, it is essential to target highly conserved regions of the viral genome and to express more than one amiRNA sequence in a single vector.

### amiRNA Design

Artificial miRNAs are molecules composed of a sequence derived from the natural pri-miRNA (primary miRNA) precursor and an siRNA (short interfering RNA) insert targeting a specific transcript. Replacing approximately 21 nt of the natural miRNA sequence with siRNA can affect the structure of the entire molecule and, consequently, how it is processed within the cell and its efficacy. There are only two tools for designing human amiRNAs: splashRNA (Pelossof et al., 2017) and shRNAI (Park et al., 2024) . Both design amiRNA molecules based solely on pri-miR30E.

In the present invention, nucleic acid molecules constituting amiRNA were unexpectedly designed using various natural pri-miRNAs to optimize the sequence and structure of the amiRNA. Existing tools do not perform thermodynamic calculations or optimize amiRNA molecules for structure and sequence. They focus solely on silencing efficiency rather than on the safety of these molecules (off-target effects).

### Technical challenges to be addressed

At the current stage of technological development, known vaccines are targeted at a single disease; there is a lack of universality in their action against multiple viruses. Meanwhile, the use of a universal vaccine allows for protective effects against multiple viruses simultaneously, without the need for multiple vaccinations. It has been demonstrated that reducing TMPRSS-2 protein levels or inhibiting its activity effectively blocks the entry of many different viruses into host cells.

State-of-the-art vaccines must be modified because viruses mutate rapidly. Therefore, the usefulness of traditional vaccines (targeting viral targets) is limited. Meanwhile, amiRNA targets the human TMPRSS-2 gene. Given that the sequence of this gene is highly conserved (low variability), the usefulness of vaccines targeting TMPRSS-2 is not limited in time. There is no need to modify them.

Furthermore, there are groups of patients for whom vaccines cannot be used because they do not produce immunity-for example, after chemotherapy-and there is no alternative for them. Meanwhile, achieving a protective effect after using amiRNA does not depend on the body's immune response. The molecular target of amiRNA is the transcript. Lowering its level results in a reduction of TMPRSS-2 protein levels in the cell and, consequently, in the inhibition of viral entry into cells. Achieving a protective effect is also possible in patients with immune deficiencies.

Currently used antiviral drugs and TMPRSS-2 inhibitors under development are administered systemically, which means they lack specificity (they work but affect multiple molecular targets) and their effects are short-lived. In contrast, amiRNA exhibits specificity of action on a single gene (no nonspecific effects on other molecular targets (off-target effects)). Very high doses of chemically modified oligonucleotides (ASO, dsRNA) are required to achieve TMPRSS-2 inhibition, whereas amiRNA is effective at low doses, and the amounts of siRNA released from it are similar to those of endogenous miRNA.

AmiRNA is also characterized by high efficacy, and to date, no one has demonstrated an amiRNA for silencing TMPRSS-2. Considering the method of administration and the duration of the effect, the inventors of this invention predict, based on scientific evidence, that: amiRNA provides long-term protection for approximately 6 months after administration to the respiratory system, e.g., in an AAV (Adeno-Associated Virus) vector, and it is possible to administer it during periods of increased incidence (fall-winter), whereas, by comparison, ASO has a short half-life and a large amount is required. Furthermore, the use of AAV as a carrier for amiRNA allows for targeting specific cells/tissues; for example, following inhalation of AAV (of the appropriate serotype), it is possible to direct the action of amiRNA only to specific cells within the respiratory system.

Another problem to be addressed is the design of effective and specific amiRNAs, since, as of now, their cleavage within the cell is unpredictable; heterogeneous products are formed, and the passenger strand is released, which may lead to undesirable, off-target effects (Galka-Marciniak et al., 2016; Guda et al., 2015; Miniarikova et al., 2016). Therefore, a new, original approach to amiRNA design is being sought, one that will allow for the optimization of target sequence selection, siRNA insert design, and pri-miRNA scaffold selection to ensure that amiRNAs are similar to endogenous pri-miRNAs in both sequence and structure. A method is sought to design a molecule that acts specifically, releases 100% of the correct strand, has a homogeneous 5' end, and effectively silences the target gene (maturing in the cell to yield a product or products released mainly from its 5' arm, homogeneous at the 5' end, specific, and effectively silencing the target gene). This was achieved by applying a new, proprietary approach to amiRNA design. It has been implemented into the miRarchitect web-based tool.

Given the shortcomings of the prior art regarding a universal yet specific approach to the prevention and/or treatment of viral diseases, the present invention has unexpectedly succeeded in resolving these technical problems by proposing anti-TMPRSS-2 that inhibits the expression of the TMPRSS-2 gene, which mediates viral diseases.

Thanks to the present invention, it is possible to provide protection for patients with immune system dysfunction, oncology patients, and transplant recipients (i.e., high-risk patients). Thus, the present invention resolves the existing technical problem regarding the lack of an immune response in a specific group of patients, as protection following the administration of amiRNA is not dependent on the host's immune response (antibody production).

Unexpectedly, the present invention has succeeded in resolving the technical problems that have existed to date regarding the design of effective, safe, and specific amiRNAs, thanks to the proposal of a new method for designing amiRNAs so that their structure and sequence resemble natural pri-miRNA molecules. Machine learning was utilized for this purpose. A new tool for designing amiRNA was created and applied, which enabled the design of highly effective and specific amiRNA based on three natural pri-miRNA precursors, namely (A) pri-miRNA-122, (B) pri-miRNA-135b, and (C) pri-miRNA-155.

Unexpectedly, the present invention has succeeded in solving the technical problem of the lack of specificity in the action of existing antiviral drugs, which cause numerous side effects. Kamostat and nafamostat, known from the prior art as TMPRSS-2 inhibitors and potential drugs against COVID-19, are not specific solely to TMPRSS-2. They also inhibit other enzymes, including serine proteases such as trypsin, prostasin, matrypsin, plasmin, kallikrein, thrombin, and C1 esterase-enzymes involved in maintaining proper homeostasis, particularly digestion, blood circulation, and coagulation. AmiRNA - act as specific inhibitors. They target a strictly defined sequence within the TMPRSS-2 transcript. The target sequence is selected with great care to avoid nonspecific effects.

It is known from the prior art that antiviral drugs (inhibitors, small-molecule compounds) and vaccines have short-lived effects (due to mutations), but by using AAV-amiRNA targeting the host protein (not the virus), it has been possible to provide long-term protection, eliminating the need to adapt the AAV-amiRNA vaccine/drug to the season (due to the slow mutation rate of TMPRSS-2).

The prior art includes International Patent Application No. WO2021206922A1 (published October 14, 2021), which relates to RNAi reagents, such as double-stranded RNA (dsRNA) reagents, targeting the TMPRSS-2 gene. The invention also relates to methods of using such RNAi reagents to inhibit the expression of the TMPRSS-2 gene and to methods of treating or preventing TMPRSS-2-dependent diseases, such as COVID-19. Although the described solution concerns RNAi technology reagents, these are molecules with completely different sequences and properties. dsRNA delivered in lipid carriers is unstable in cells, and thus does not provide a long-lasting effect after a single administration. It is not possible to deliver them specifically to selected cell types or tissues, thereby increasing the risk of side effects. Lipid carriers and chemical modifications of dsRNA can trigger an immune response. Exogenous dsRNA is a known pathogen-associated molecular pattern (PAMP) and can trigger an interferon response through the activation of cellular dsRNA sensors such as RIGI, PKR, OAS, MDA5, and TLR3 (Olejniczak et al., 2010). All these characteristics mean that, unlike amiRNA, the described solution does not ensure safety, specificity of action, or a long-lasting effect.

Furthermore, international patent application No. WO2021248027A1 (published 2021-12-09) is known in the prior art, which relates to compounds, compositions, and methods for inhibiting angiotensin-converting enzyme 2 (ACE2) and transmembrane serine protease 2 (TMPRSS-2) in a cell, such as a lung cell, to inhibit severe acute respiratory syndrome coronavirus 2 (SARS-CoV-2) and prevent or treat the associated disease, coronavirus disease 2019 (COVID-19). This solution relates to modified antisense oligonucleotides (ASOs), which differ significantly from amiRNA in terms of structure, mechanism of action, and delivery method, and do not provide a long-lasting silencing effect after a single administration.

Also known is international patent application No. WO2019222413A1 (published November 21, 2019), which relates to nucleic acids and recombinant adenovirus-associated viruses (rAAV) designed to express a transgene comprising an inhibitory nucleic acid (e.g., amiRNA) containing a scaffold consisting of a mouse pri-miRNA and a guide strand that targets a human target gene. The use of mouse miRNA precursors as carriers for siRNA is a significant difference from the present application. These sequences may contain important structural features and sequence motifs that influence their expression levels and cellular processing. Additionally, an artificial scaffold for siRNA was developed in the form of a modified mouse pri-miR33, which improved the integrity of the AAV genome. This solution describes molecules targeting the SOD1 and PC1 genes and a method for treating amyotrophic lateral sclerosis (ALS).

Finally, there is also the international patent application No. WO2022078569A1 (published April 21, 2022), which relates to artificial nucleic acids for targeted editing of target RNA. In particular, this invention provides synthetic nucleic acids capable of targeted editing of endogenous transcripts through the use of endogenous deaminase. Furthermore, this invention provides artificial nucleic acids for targeted editing of target RNA, which ensure increased editing specificity to avoid unwanted off-target editing. The invention also encompasses a method for producing the aforementioned artificial nucleic acid, wherein at least some steps of the method are computer-implemented or computer-executed.

Despite existing solutions describing methods for silencing the expression of the gene encoding the TMPRSS-2 protein, there remains a constant need to provide a solution capable of specifically, safely, and long-term inhibiting the expression of the gene encoding the TMPRSS-2 protein involved in viral diseases.

### OBJECTIVE OF THE INVENTION

The objective of the present invention is to inhibit the expression of the gene encoding the TMPRSS-2 protein, which plays a role in viral diseases, by targeting specific sequences in the TMPRSS-2 transcript using RNA interference (RNAi) reagents. The solution according to the invention comprises artificial miRNA (amiRNA) inhibitors with a structure and sequence resembling natural pri-miRNA molecules, consisting of a TMPRSS-2 gene-specific siRNA insert and a scaffold based on natural precursor miRNA (pri-miRNA). Their intracellular expression enables a continuous supply of siRNA molecules, thereby prolonging their action. AmiRNA precursors are incorporated into the endogenous miRNA biogenesis pathway and undergo a two-step cellular processing by the enzymes Drosha and Dicer. AmiRNAs stand out from other RNA interference tools known in the prior art due to their high specificity of gene silencing.

The invention may find application in the therapy and/or prevention of TMPRSS-2-dependent viral diseases, demonstrating high efficacy, specificity, and versatility (activity against multiple viruses), long-lasting protection without the need to design and synthesize new molecules in subsequent seasons, and which also provides protection for patients with immune system dysfunction, such as cancer patients and transplant recipients (i.e., high-risk patients).

This objective was achieved through the invention defined in the attached patent claims, with advantageous variants thereof defined in the dependent claims.

### Advantages of the invention

The nucleic acid molecule of the present invention silences the expression of the gene encoding the transmembrane serine protease 2 (TMPRSS-2) in a cell by reducing the levels of the TMPRSS-2 transcript and protein. According to the present invention, nucleic acid molecules (anti-TMPRSS-2 amiRNAs of the present invention) reduce the level of TMPRSS-2 protein in cells, thereby limiting the ability of TMPRSS-2-dependent viruses to enter cells and thus limiting the progression of viral infections.

The nucleic acid molecule of the present invention (anti-TMPRSS-2 amiRNA of the present invention) is universal and therefore provides a protective/therapeutic effect against many viral diseases, such as COVID-19 (SARS-CoV-2 virus), SARS (SARS-CoV), MERS (MERS-CoV), influenza (e.g., influenza A viruses: H1N1, H3N2, influenza B), parainfluenza (parainfluenza viruses PIV-1 and PIV-3), infections caused by respiratory syncytial virus (RSV), and Zika fever (Zika virus).

Artificial amiRNAs targeting the TMPRSS-2 transcript, delivered to cells e.g., in the form of rAAV, inhibit the expression of the gene encoding the TMPRSS-2 protein, thereby reducing TMPRSS-2 protein levels and thus inhibiting viral entry into host cells. Their use in humans may serve as an effective preventive measure against viral infections in high-risk populations and achieve a therapeutic effect in patients affected by TMPRSS-2-dependent viral diseases.

It is generally known in the prior art that the effect of anti-TMPRSS-2 amiRNA is long-lasting and lasts approximately six months after a single administration in an AAV vector (Muhuri et al., 2022; Tang et al., 2024) .

Existing vaccines must be modified because viruses mutate, whereas the amiRNA sequence according to the present invention (constant amiRNA sequence) does not need to be modified, as it targets the human TMPRSS-2 gene (negligible variability). Thanks to resistance/insensitivity to the rapid mutation rate of viruses, there is no need to update the vaccine/drug every season.

Currently used state-of-the-art antiviral drugs and TMPRSS-2 inhibitors under development are administered systemically, resulting in low specificity of action; in contrast, anti-TMPRSS-2 amiRNAs-TMPRSS-2 according to the present invention are suitable for administration, e.g., to the respiratory system, e.g., via an AAV virus with a serotype specific to the cells of that system, and will exhibit specificity of action in the target tissue and toward a single gene (no off-target effects).

Furthermore, with regard to the anti-TMPRSS-2 amiRNA of the present invention:
- the therapeutic/protective effect is the result of a reduction in host protein levels,
- the therapeutic/protective effect is achieved independently of the body's immune response,
- it can be administered to patients with immune system dysfunction,
- convenient administration routes are ensured: intranasally, by inhalation, but also enterally, such as orally, or parenterally, such as by intravenous, intramuscular, or subcutaneous injection, etc.
- a convenient and safe form of administration is provided in the form of vectors, e.g., AAV.

A method for obtaining amiRNA not covered by the invention, which utilizes machine learning-based design and assumes that artificial miRNAs must be as similar as possible to natural pri-miRNAs, allows for the rapid design of efficient and specific amiRNA molecules that can be used in the treatment of human diseases. A unique advantage of the amiRNA design tool (miRarchitect) is the use of machine learning to optimize target sequence selection, siRNA insert design, and pri-miRNA scaffold selection, ensuring that the resulting amiRNAs are highly similar to endogenous pri-miRNAs in both sequence and structure (this is an original approach to amiRNA design). Additionally, miRarchitect allows for the selection of safe and specific molecules that do not pose a risk of off-target effects. Using miRarchitect within the scope of this invention, an anti-TMPRSS-2 amiRNA molecule was designed that has no potential off-target interaction regions, releases 100% of the correct strand, has a homogeneous 5' end, and effectively silences TMPRSS-2.

### BRIEF DESCRIPTION OF THE FIGURES

The subject matter of the invention is illustrated in the embodiments, with reference to the accompanying drawings, in which:
- **FIG. 1**: shows a schematic of the TMPRSS-2 transcript with indicated target sites recognized by nucleic acid molecules according to the present invention (amiRNA according to the present invention).
- **FIG. 2**: shows the results of an *in vitro* analysis of the efficacy of anti-TMPRSS-2 amiRNAs, which are the subject of the invention. The graph shows the results of luciferase reporter silencing by anti-TMPRSS-2 amiRNAs according to the present invention.
- **FIG. 3**: shows the results of an analysis of the TMPRSS-2 transcript level after application of the amiRNA inhibitors of the invention.
- **FIG. 4**: shows the results of an analysis of TMPRSS-2 protein levels following the use of the anti-TMPRSS-2 amiRNA inhibitors of the invention.
- **FIG. 5**: shows the maturation products of amiRNA precursors according to the present invention in HEK293T cells, based on small RNA sequencing (NGS) analysis. For simplicity, the figure shows amiRNA molecules with truncated 5' and 3' flanking sequences.
The amiRNAs of the present invention were designed and synthesized using the following templates**: (A)** pri-miRNA-122, **(B)** pri-miRNA-135b, and **(C)** pri-miRNA-155, were introduced into HEK293T cells, where, following a two-step processing by the RNases Drosha and Dicer, they were cleaved into short siRNAs composed of a guide and a passenger strand. The products generated from individual amiRNA precursors are shown, along with their percentage representation. Arrows indicate the cleavage sites of amiRNA precursors with Drosha.

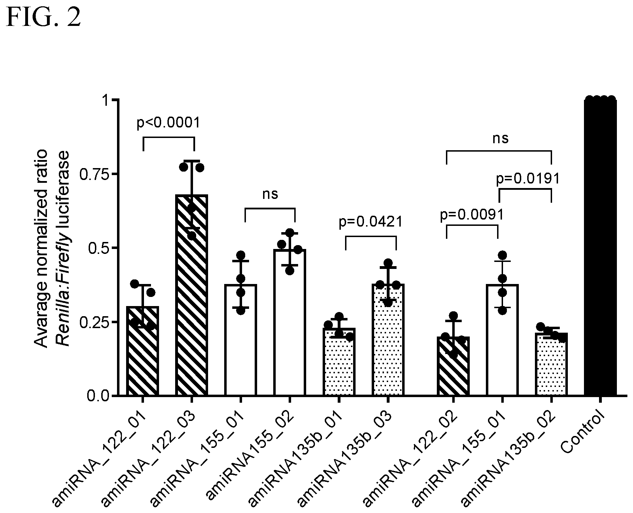

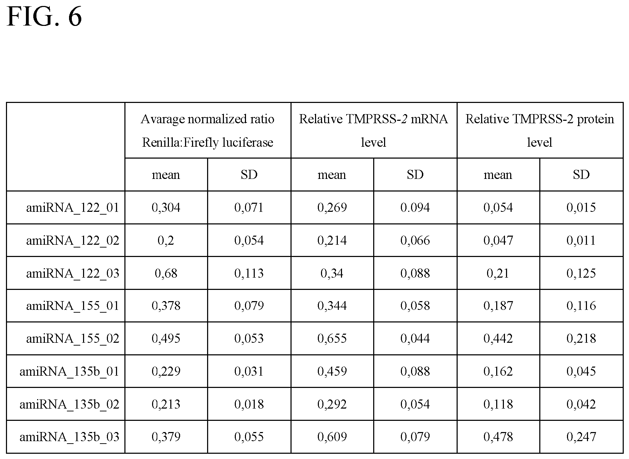

- **FIG. 6**: presents a summary of the results of the analysis of anti-TMPRSS-2 amiRNA activity according to the present invention in HEK293T cells. The table shows the mean values of the luciferase reporter, TMPRSS-2 transcript and protein levels, and standard deviations. Statistical significance is presented in FIG. 2, 3, and 4.

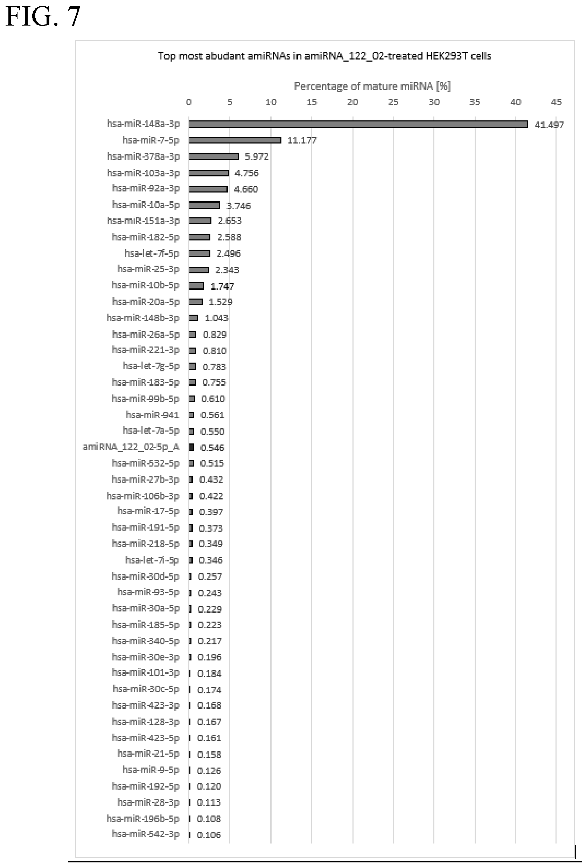

- **FIG. 7**: shows the level of the sense strand of amiRNA_122_02 according to the present invention in HEK293T cells compared to the level of endogenous miRNAs. The analysis was performed based on the measurement of read counts in small RNA sequencing following transfection of cells with the pcDNA3.1_amiRNA_122_02 expression plasmid. The level of the sense strand released from amiRNA_122_02 according to the present invention constitutes 0.546% of the level of the most abundant endogenous miRNAs in the cell (Top 50).

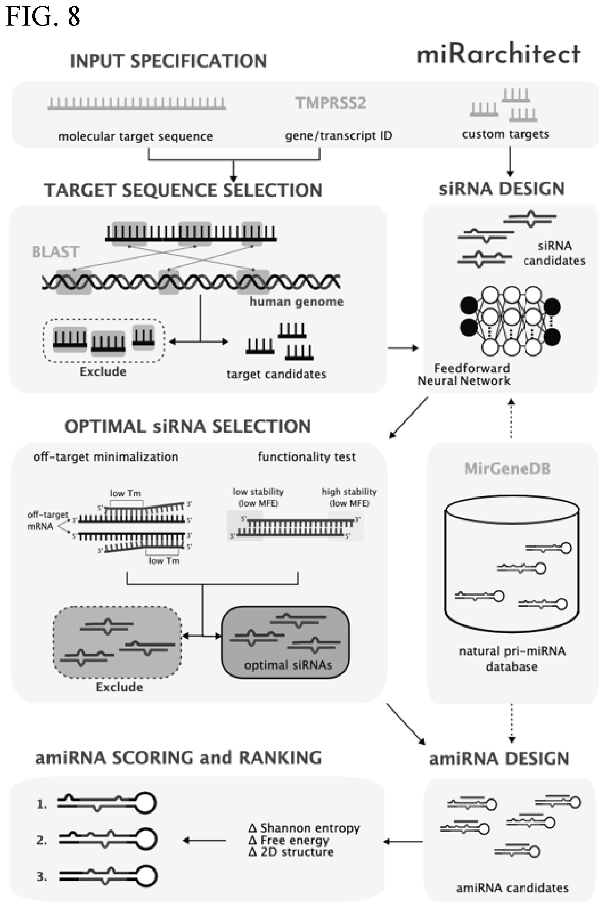

- **FIG. 8**: schematically illustrates the method (not covered by the invention) of designing artificial miRNAs according to the present invention that target the TMPRSS-2 coding sequence.

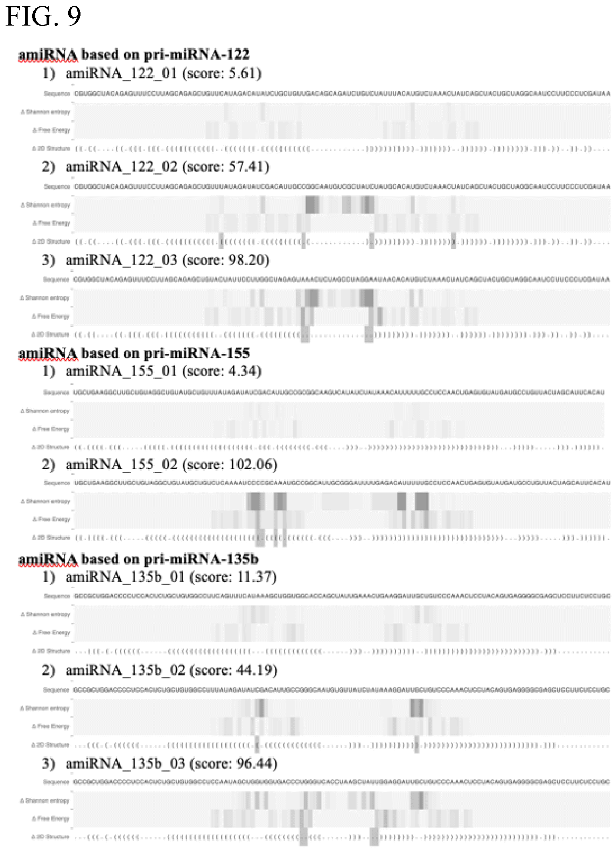

- **FIG.** 9: shows a comparison of the thermodynamic parameters and secondary structure of anti-TMPRSS-2 amiRNA candidates according to the present invention with their carrier pri-miRNAs.

### SUMMARY OF THE INVENTION

The subject of the present invention is a nucleic acid molecule for silencing the expression of the gene encoding transmembrane serine protease 2 (TMPRSS-2) in a cell, forming a hairpin structure, comprising in the 5' to 3' direction:
- a 5' flanking sequence,
- a guide strand,
- a terminal loop,
- a passenger strand,
- a 3' flanking sequence,
wherein the terminal loop connects the guide strand and the passenger strand, which strands form a duplex of the guide strand and the passenger strand located between the terminal loop and the 5' and 3' flanking sequences, characterized in that said duplex comprises said guide strand having a nucleotide sequence selected from the sequences with ID numbers: 2, 5, 8, 12, 15, and 19, or a nucleotide sequence having at least 90% identity to a nucleotide sequence selected from the nucleotide sequences with ID numbers: 2, 5, 8, 12, 15, and 19, wherein said guide strand is complementary to the mRNA encoding the TMPRSS-2 protein, wherein said passenger strand is at least 60% complementary to said guide strand, wherein the duplex of the guide strand and the passenger strand is located between the terminal loop of human pri-miRNA and the 5' and 3' flanking sequences of the human pri-miRNA, and wherein the nucleic acid molecule, upon introduction into a cell expressing the gene encoding the TMPRSS-2 protein, silences the expression of that gene.

Preferably, the duplex comprises a short interfering RNA (siRNA) consisting of a guide strand and a passenger strand, each of which has a length of 20 to 24 nucleotides.

Preferably, the duplex comprises a short interfering RNA (siRNA) consisting of a guide strand and a passenger strand, each of which is 22 nucleotides in length.

Preferably, the passenger strand has a nucleotide sequence selected from the sequences with accession numbers 24 through 31.

Preferably, the passenger strand is at least 77% complementary to the guide strand.

Preferably, the template strand is fully complementary to the mRNA encoding the TMPRSS-2 protein having a sequence selected from the sequences with ID numbers: 3, 6, 9, 13, 16, and 20, or is at least 90% complementary to the mRNA encoding the TMPRSS-2 protein with a sequence selected from the sequences with ID numbers: 3, 6, 9, 13, 16, and 20.

Preferably, the nucleic acid molecule comprises a terminal loop, a 5' flanking sequence, and a 3' flanking sequence of a human pri-miRNA selected from pri-miRNA-122, pri-miRNA-155, pri-miRNA-135b, pri-miRNA-21, and pri-miRNA-30a, preferably pri-miRNA selected from pri-miRNA-122 or pri-miRNA-155, wherein the terminal loop, the 5' flanking sequence, and the 3' flanking sequence of the human pri-miRNA are truncated or are not truncated.

Preferably, the terminal loop comprises from 4 to 20 nucleotides; more preferably, the terminal loop comprises from 14 to 17 nucleotides.

Preferably, the terminal loop has a sequence selected from the sequences having ID Nos. 21-23.

Preferably, the nucleic acid molecule constitutes an artificial microRNA (amiRNA).

The present invention further relates to an expression cassette for silencing the expression of the gene encoding the TMPRSS-2 protein, comprising a regulated or constitutive promoter, characterized in that said promoter is functionally linked to the sequence of the nucleic acid molecule as defined above.

Preferably, the promoter is RNA polymerase II (RNA pol II) or RNA polymerase III (RNA pol III), wherein the RNA pol II promoter is preferably selected from the cytomegalovirus (CMV) promoter or the CAG promoter consisting of a CMV enhancer and a chicken beta-actin promoter.

Preferably, the expression cassette further comprises a polyadenylation signal selected from the human growth hormone polyadenylation signal (hGH poly A), the late polyadenylation signal of simian virus 40 (SV40L poly A), or short poly A; and a post-transcriptional regulatory element of the marmot hepatitis virus (WPRE).

The present invention also relates to an expression vector for silencing the expression of the gene encoding the TMPRSS-2 protein, characterized in that it comprises a nucleic acid molecule as defined above or an expression cassette as defined above.

Preferably, the expression vector is a vector selected from among an adeno-associated virus (AAV), a lentivirus, an adenovirus, a plasmid, an anellavirus, a non-viral DNA vector, a minimal DNA vector, and a non-integrating lentivirus.

The present invention also relates to a pharmaceutical composition for silencing the expression of the gene encoding the TMPRSS-2 protein in the body, characterized in that it comprises at least one nucleic acid molecule as defined above, or an expression cassette as defined above, or a vector as defined above, and a pharmaceutically acceptable carrier.

Preferably, the pharmaceutical composition comprises a nucleic acid molecule comprising a duplex of a guide strand and a passenger strand, wherein the guide strand has a nucleotide sequence selected from the sequences identified by numbers 2, 5, 8, 12, 15, and 19, or a nucleotide sequence having at least 90% identity to the nucleotide sequence selected from the nucleotide sequences with ID numbers: 2, 5, 8, 12, 15, and 19, wherein said guide strand is complementary to the mRNA encoding the TMPRSS-2 protein, wherein said passenger strand is at least 60% complementary to said guide strand, preferably said passenger strand is 77% complementary to said guide strand, and wherein the duplex of the guide strand and the passenger strand is located between the terminal loop of the human pri-miRNA and the 5' and 3' flanking sequences of the human pri-miRNA, wherein, preferably, the human pri-miRNA is selected from pri-miRNA-122, pri-miRNA-155, pri-miRNA-135b, pri-miRNA-21, pri-miRNA-30a, and more preferably the human pri-miRNA is selected from pri-miRNA-122 or pri-miRNA-155.

Preferably, the pharmaceutical composition comprises a nucleic acid molecule comprising a duplex of a guide strand and a passenger strand, wherein the guide strand is fully complementary to an mRNA encoding the TMPRSS-2 protein having a sequence selected from the sequences with ID Nos. 3, 6, 9, 13, 16, and 20, or is at least 90% complementary to the mRNA encoding the TMPRSS-2 protein having a sequence selected from the sequences with ID numbers: 3, 6, 9, 13, 16, and 20.

Preferably, the pharmaceutical composition comprises a nucleic acid molecule containing a passenger strand, wherein the passenger strand has a nucleotide sequence selected from the sequences with ID numbers: 24 to 31.

Preferably, the pharmaceutically acceptable carrier is selected from a stabilizing buffer, a protective protein, a stability-enhancing agent, and a means for *in vivo* administration; wherein the stabilizing buffer is selected from phosphate-buffered saline (PBS), Tris-HCl, HEPES, NaCl, MgCl₂, KCl, and protective sugars such as sucrose and trehalose; wherein the protective protein is human serum albumin (HSA); wherein the stability-enhancing agent is selected from Polysorbate and polyethylene glycol (PEG), preferably Polysorbate 80; wherein the *in vivo* delivery agent is selected from lipids, nanoparticles, hydrogel, and microspheres.

The subject of the present invention is also a nucleic acid molecule as defined above for use in therapy.

The subject of the present invention is also a nucleic acid molecule as defined above for use in the treatment and/or prevention of a viral disease dependent on transmembrane serine protease 2 (TMPRSS-2).

Preferably, the viral disease dependent on type 2 transmembrane serine protease is a disease selected from severe acute respiratory syndrome (SARS), Middle East respiratory syndrome (MERS), coronavirus disease 2019 (COVID-19), influenza, parainfluenza, respiratory syncytial virus (RSV) infection, and Zika fever.

The present invention also relates to an *in vitro* method for silencing the expression of the gene encoding the transmembrane serine protease 2 (TMPRSS-2) protein in a cell, characterized in that it comprises the steps in which:
(a) a nucleic acid molecule as defined above, or an expression cassette as defined above, or a vector as defined above is introduced into a cell, and
(b) the cell containing the introduced nucleic acid molecule or expression cassette or vector from step (a) is maintained under conditions and for a time sufficient to achieve silencing of the expression of the gene encoding the TMPRSS-2 protein in the cell.

Preferably, in the method of the invention, in step (a), the nucleic acid molecule comprises a duplex of a guide strand and a passenger strand, wherein the guide strand has a nucleotide sequence selected from the sequences with ID Nos. 2, 5, 8, 12, 15, and 19, or a nucleotide sequence having at least 90% identity to a nucleotide sequence selected from the nucleotide sequences with ID numbers: 2, 5, 8, 12, 15, and 19, wherein said guide strand is complementary to the mRNA encoding the TMPRSS-2 protein, and wherein said passenger strand is at least 60% complementary to said guide strand, preferably said passenger strand is 77% complementary to said guide strand, and wherein the duplex of the guide strand and the passenger strand is located between the terminal loop of the human pri-miRNA and the 5' and 3' flanking sequences of the human pri-miRNA, wherein, preferably, the human pri-miRNA is selected from pri-miRNA-122, pri-miRNA-155, pri-miRNA-135b, pri-miRNA-21, pri-miRNA-30a, and more preferably the human pri-miRNA is selected from pri-miRNA-122 or pri-miRNA-155.

Preferably, in the method according to the invention, in step a), the nucleic acid molecule comprises a duplex of a guide strand and a passenger strand, wherein the guide strand is fully complementary to mRNA encoding the TMPRSS-2 protein having a sequence selected from sequences with ID Nos: 3, 6, 9, 13, 16, and 20, or is at least 90% complementary to the mRNA encoding the TMPRSS-2 protein having a sequence selected from the sequences with ID Nos: 3, 6, 9, 13, 16, and 20.

Preferably, in the method according to the invention, in step a), the nucleic acid molecule comprises a passenger strand, wherein the passenger strand has a nucleotide sequence selected from the sequences with ID Nos: 24 to 31.

Preferably, in the method according to the invention, silencing of the expression of the gene encoding the TMPRSS-2 protein is achieved using an RNA interference (RNAi) method based on artificial microRNA (amiRNA).

### DETAILED DESCRIPTION OF THE INVENTION

The subject matter of the present invention is described in detail below with reference to the accompanying figures and embodiments. The present invention is not limited to the specific embodiments described herein.

In general, "artificial miRNAs (amiRNAs)" consist of a therapeutic-target-specific siRNA insert (guide strand and passenger strand) and a scaffold based on a natural precursor miRNA (in this invention, pri-miRNA). Their intracellular expression enables the sustained delivery of siRNA molecules, thereby prolonging their effect. amiRNA precursors are incorporated into the endogenous miRNA biogenesis pathway and undergo a two-step cellular processing by the enzymes Drosha and Dicer. Artificial miRNAs stand out from other RNA interference tools known in the prior art due to their high specificity in gene silencing. Their success in clinical trials demonstrates that they are an effective and safe therapeutic tool for a wide spectrum of human diseases (Kotowska-Zimmer et al. 2021).

The creation of an artificial miRNA involves inserting a siRNA sequence into the backbone of a natural precursor miRNA (pri-miRNA) in such a way that the native biogenesis process leads to the formation of the expected homogeneous pool of effector molecules (siRNA pool).

The term "nucleic acid molecule" as used herein refers to a ribonucleic acid (RNA) molecule or a corresponding deoxyribonucleic acid (DNA) molecule. In this patent specification, this term refers to the artificial miRNA molecules of the invention. The term "human pri-miRNA" refers to a natural precursor of human miRNA. "MicroRNA" or "miRNA" is a small non-coding RNA molecule capable of mediating post-transcriptional gene silencing. Its sequence is located in various regions of the genome. It is typically produced by transcription involving RNA polymerase II (RNA Pol II) and forms a primary miRNA (pri-miRNA) with a hairpin structure. It is enzymatically processed (e.g., by Drosha, DGCR8, Pasha, etc.) into a shorter pre-miRNA, which is exported to the cytoplasm and further enzymatically processed (by Dicer, TRBP, PACT, etc.). This processing results in a miRNA/miRNA* duplex, from which one strand is removed, and the resulting single-stranded mature miRNA molecule is loaded into the RNA-induced silencing complex (RISC). Typically, the mature miRNA molecule is approximately 19 to 30 base pairs in length.

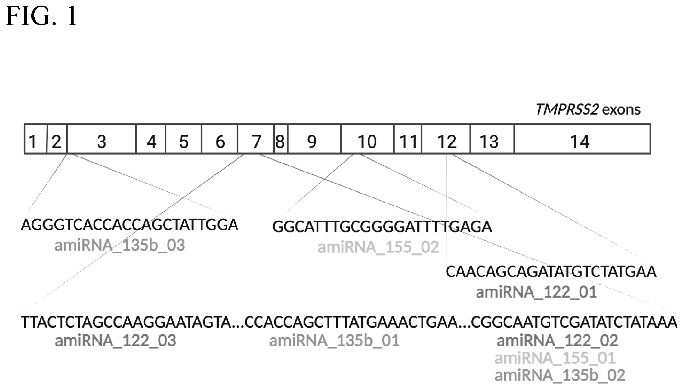

In light of the described prior art, the present invention aims to overcome the indicated drawbacks of current antiviral therapies/vaccines and to provide an alternative approach based on artificial miRNAs targeting the TMPRSS-2 transcript (FIG. 1), advantageously delivered to cells in the form of vectors such as, for example, rAAV.

Referring to FIG. 1, a schematic of the TMPRSS-2 transcript is shown with indicated target sites recognized by the amiRNA of the present invention.

### amiRNA molecules according to the present invention targeting the human TMPRSS-2 transcript

The subject of the present invention is a nucleic acid molecule for silencing the expression of the gene encoding the transmembrane serine protease type 2 (TMPRSS-2) protein in a cell, forming a hairpin-like structure containing, in the 5' to 3' direction:
- the 5' flanking sequence,
- guide strand,
- terminal loop,
- passenger strand,
- the 3' flanking sequence,
wherein the terminal loop connects the guide strand and the passenger strand, which strands form a guide-passenger strand duplex located between the terminal loop and the 5' and 3' flanking sequences. The aforementioned duplex of the guide strand and the passenger strand is located between the terminal loop of the human pri-miRNA and the 5' and 3' flanking sequences of the human pri-miRNA.

The present invention discloses sequences of amiRNA precursor molecules targeting the human TMPRSS-2 transcript (Seq ID No.: 1, Seq ID No.: 4, Seq ID No.: 7, Seq ID No.: 10, Seq ID No.: 11, SEQ ID No: 14, SEQ ID No: 17, and SEQ ID No: 18). They consist of a therapeutic-target-specific short interfering RNA (siRNA) insert, i.e., the guide strand and the passenger strand, and a scaffold, i.e., the remaining part of the nucleic acid molecule (3' and 5' flanking sequences and the terminal loop) based on natural precursor miRNAs (pri-miRNA, i.e., pri-miRNA-122, pri-miRNA-155, or pri-miR-135b).

The therapeutic target is understood to be a complementary sequence in the TMPRSS-2 transcript, recognized by the siRNA guide strand. The siRNA guide strand is fully complementary to the TMPRSS-2 transcript or is at least 90% complementary to the TMPRSS-2 transcript. Methods for determining complementarity, including determining the percentage of complementarity, are known in the art.

Preferably, the pri-miRNA used as a scaffold yields homogeneous products from the 5' end, e.g., pri-miRNA-122, pri-miRNA-155. Preferably, the amiRNA is as similar as possible to the natural pri-miRNA on which it is based in terms of three parameters: free energy (G), Shannon entropy, and secondary structure. The amiRNA design tool miRarchitect, developed by the inventors of the present invention, enables the design of such molecules. Preferably, neither of the siRNA strands (the guide and the passenger) exhibits complementarity to any other transcript other than TMPRSS-2, i.e., it is not a source of off-target activity.

### amiRNA molecules (precursors)-nucleic acid molecules according to the present invention targeting the TMPRSS-2

According to the present invention, amiRNA_122_01 targeting the TMPRSS-2 sequence has the sequence shown in SEQ ID No: 1, with a guide strand as shown in SEQ ID No: 2 and a passenger strand as shown in SEQ ID No: 24, fully complementary to the sequence in Sequence ID No.: 3 located within the TMPRSS-2 transcript and capable of specifically reducing the level of the transcript, and consequently the TMPRSS-2 protein.

Similarly, for the other amiRNAs according to this invention:

| amiRNA targeting the TMPRSS-2 sequence | Seq. ID No. | | | |
|---|---|---|---|---|
| | amiRNA | amiRNA guide strand | Sequence located within the TMPRSS-2 transcript to which the amiRNA sense strand is complementary | amiRNA passenger strand |
| amiRNA_122_01 | 1 | 2 | 3 | 24 |
| amiRNA_122_02 | 4 | 5 | 6 | 25 |
| amiRNA_122_03 | 7 | 8 | 9 | 26 |
| amiRNA_155_01 | 10 | 5 | 6 | 30 |
| amiRNA_155_02 | 11 | 12 | 13 | 27 |
| amiRNA_135b_01 | 14 | 15 | 16 | 28 |
| amiRNA_135b_02 | 17 | 5 | 6 | 31 |
| amiRNA_135b_03 | 18 | 19 | 20 | 29 |

The sequences referred to above are presented in Table 1 at the end of the patent specification and in the attached sequence listings.

According to the present invention, the passenger strand has a nucleotide sequence selected from sequences with ID numbers 24 to 31 (Table 1) and is at least 77% complementary to the guide strand (at least 17 nucleotides of the duplex out of 22 nucleotides are complementary).

In other embodiments, the aforementioned template strand has a nucleotide sequence that is at least 90% identical to the nucleotide sequence selected from the sequences with ID numbers: 2, 5, 8, 12, 15, and 19 and is 20 to 24 nucleotides long. The passenger strand is then at least 60% complementary to the guide strand.

In other embodiments, the pri-miRNAs may also be other human pri-miRNAs, such as pri-miRNA-21 or pri-miRNA-30a.

### Efficiency of reducing TMPRSS-2 transcript and protein levels in HEK293T cells by nucleic acid molecules (amiRNA) according to the present invention

The efficiency of reducing TMPRSS-2 transcript and protein levels in HEK293T cells transfected with a plasmid encoding exogenous TMPRSS-2 and plasmids encoding individual amiRNAs according to the invention, 24 hours after plasmid administration, ranges from:
a. 50% (for amiRNA_155_02 according to the invention) to as much as 80% (amiRNA_122_02 according to the invention) - using a luciferase reporter system,
b. 35% (amiRNA_155_02 according to the invention) to 79% (amiRNA_122_02 according to the invention) - at the transcript level,
c. 40% (amiRNA_155_02 of the invention) to 95% (amiRNA_122_01 of the invention and amiRNA_122_02 of the invention) at the protein level.

The efficiency, standard deviations, and statistical significance of the changes for individual amiRNAs in the three cellular models are presented in FIG. 2, 3, 4, 6. TAB. 1

### Maturation products of amiRNA precursors

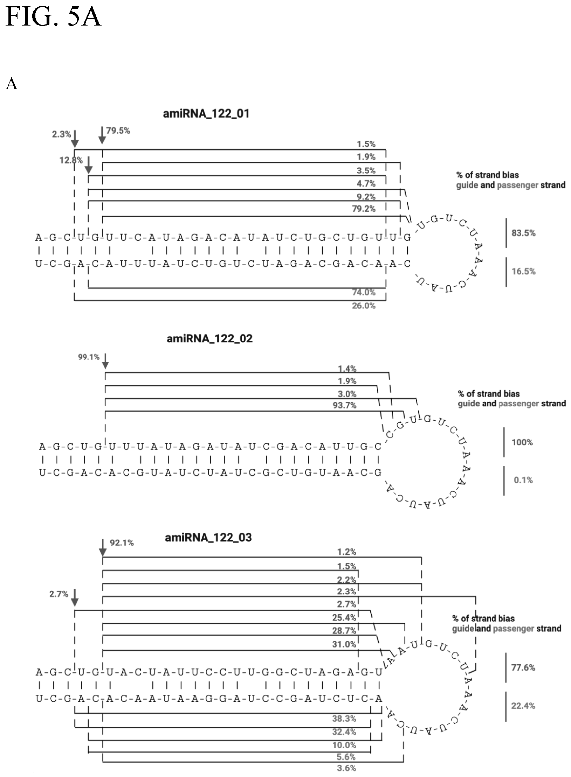

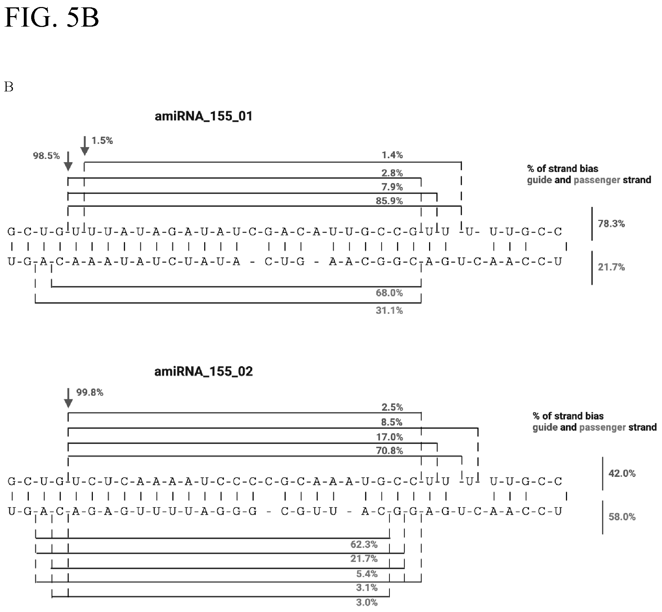

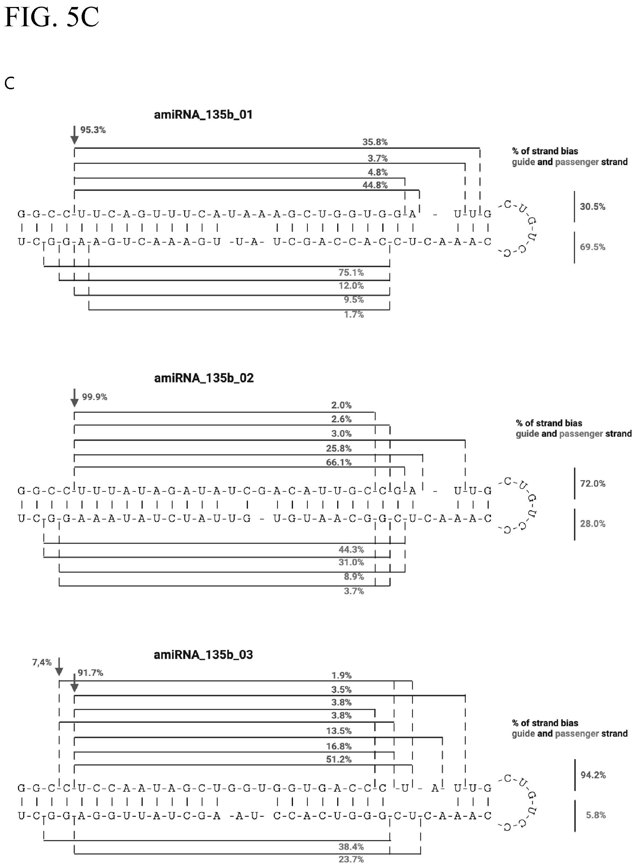

The maturation products of amiRNA precursors according to the present invention were identified in HEK293T cells (FIG. 5).

Referring to FIG. 5, the maturation products of amiRNA precursors according to the present invention in HEK293T cells are shown, based on small RNA sequencing (NGS) analysis. amiRNA designed and synthesized using the templates (FIG. 5A) pri-miRNA-122, (FIG. 5B) pri-miRNA-135b, and (FIG. 5C) pri-miRNA-155 were introduced into HEK293T cells, where, as a result of two-step processing by the RNases Drosha and Dicer, they were cleaved into short siRNAs, consisting of a guide strand and a passenger strand. The products generated from individual amiRNA precursors are shown, along with their percentage representation. Arrows indicate the cleavage sites of the amiRNA precursors involving Drosha.

All amiRNA precursors designed according to the present invention are cleaved very precisely in cells with the participation of Drosha. All products derived from the 5' arm may be a source of activity, i.e., they may be responsible for silencing the TMPRSS-2 transcript and protein. None of the products exhibits significant complementarity to transcripts other than TMPRSS-2 and is not a source of off-target activity.

### Method for designing effective and specific amiRNAs

Using the bioinformatics tool miRarchitect (https://rnadrug.ibch.poznan.pl/mirarchitect), amiRNAs targeting the TMPRSS-2 transcript were designed. Referring to FIG. 8, a schematic representation is provided of how artificial miRNAs according to the present invention targeting the TMPRSS-2 coding sequence were designed.

The high efficacy of these molecules at the transcript and protein levels was confirmed in two reporter assays, and their cellular processing was detected using NGS (small RNA sequencing). In these studies, the amiRNAs were introduced into human HEK293T cells in the form of an expression plasmid encoding the amiRNA gene under the CMV promoter. The amiRNA gene was synthesized by GeneScript and cloned into the pcDNA3.1/Hygro(+) plasmid at the HindIII/XhoI restriction sites (Thermo Fisher Scientific).

The design method, which is not covered by this invention, is a fully automated approach to designing amiRNAs according to this invention. During the design process, the sequence, structure, and thermodynamics of siRNA duplexes-i.e., duplexes of the guide and passenger strands-and amiRNA candidates (consisting of a guide-passenger strand duplex and a scaffold derived from a natural miRNA). Ultimately, those amiRNAs are selected whose structure and thermodynamic parameters are most similar to the natural pri-miRNA on which the amiRNA was designed.

To optimize the design process, a neural network dedicated to siRNA design was created and trained. A key advantage of this approach is also the minimization of potential non-specific interactions of the amiRNA, through the careful selection of target sequences for the amiRNA and the thermodynamic parameters and sequences of both strands (guide and passenger) of the siRNA insert.

The amiRNA design presented in this invention comprises 5 steps:
(1) providing an input sequence containing the target sequence for the amiRNA;
(2) identifying the target sequence within the input sequence from step (1);
(3) neural network-assisted design of an siRNA insert (guide strand and passenger strand) complementary to the target sequence from step (2), wherein the siRNA insert consists of a 20-22 nt duplex and 2 unpaired nucleotides at each 3' end, and its preliminary evaluation,
(4) designing amiRNA candidates based on natural miRNA, preferably pri-miRNA-21, pri-miRNA-30a, pri-miRNA-122, pri-miRNA-155, or pri-miRNA-135b, serving as a scaffold for the siRNA insert (guide strand and passenger strand) from step (3);
(5) evaluating the structure, free energy, and Shannon entropy for individual nucleotide positions of the amiRNA candidates obtained in step (4) and creating a ranked list of them, such that the candidates whose secondary structure, free energy, and Shannon entropy are most similar to the natural pri-miRNA based on which the amiRNA was designed in step (4), and selecting the amiRNA candidate most similar to the natural miRNA.

In step (1), the input data for design can be: a DNA/RNA sequence, or the name or number of a transcript/gene. In the case of a gene, the program generates a list of corresponding transcripts, from which the user must select one. The sequence is then compared using BLAST against sequences contained in biological databases. The sequence with the longest matched fragment is defined as the input sequence (molecular target), i.e., the one for which amiRNAs are designed in subsequent steps. In step (1), the TMPRSS-2 transcript sequence (GenBank accession number: NM_005656, transcript: ENST00000332149) was used for amiRNA design.

In step (2), within the input sequence from the aforementioned step (1), the program selects 24-nucleotide fragments that simultaneously meet the following criteria: (i) their GC content falls within the range of 30-65%, (ii) they do not contain 9-nucleotide or longer GC runs, (iii) they do not exhibit high homology with other sequences deposited in biological databases. In this step, target sequences for siRNA were selected, which consisted of 24-nucleotide fragments of the TMPRSS-2 transcript.

In step (3), the design of the guide strand and the passenger strand (i.e., the siRNA insert) complementary to the target sequence from step (2) is performed using a neural network created by the inventors of the present invention. The guide strand (complementary to the target sequence from step (2)) and the passenger strand (with permissible mismatches to the guide strand, provided that the passenger strand is at least 60% complementary to the aforementioned guide strand) are designed sequentially. As a result, the miRarchitect algorithm generates a list of siRNA-20-22-nt duplexes with 2-nt unpaired ends at the 3' termini. Next, the thermodynamic parameters of the duplex and the individual strands are evaluated to eliminate non-functional siRNAs and those that may interact nonspecifically with other sequences. In particular, siRNAs with varying terminal stability are selected, with the guide strand having lower 5' terminal stability. In addition, siRNAs are selected whose melting temperature within the seed region of the guide or passenger strand (seed region-the region within the guide and passenger strands encompassing nucleotides 2-7 from the 5' end of each strand) is lower than 21.5°C, which is intended to ensure the specificity of their action. For the siRNAs that have been designed and evaluated positively, the next step involves designing amiRNAs.

In step (4), for each siRNA, the program selects an appropriate scaffold/carrier from among natural human pri-miRNAs that yield homogeneous products from the 5' end, preferably from pri-miRNA-21, pri-miRNA-30a, pri-miRNA-122, pri-miRNA-155, or pri-miRNA-135b, preferably pri-miRNA-122 or pri-miRNA-155. As a result, amiRNA candidates according to the present invention are obtained.

In step (5), the structure and thermodynamic parameters-including free energy and Shannon entropy-are evaluated for each nucleotide position in the amiRNA candidates generated in step (4). Ultimately, the amiRNAs selected (those at the top of the ranking list) are those whose structure and thermodynamic parameters are most similar to the natural pri-miRNA on which the amiRNA was designed.

With reference to FIG. 9, differences in free energy (Δ free energy) and differences in Shannon entropy (Δ Shannon entropy) for individual positions (nucleotides) are presented in the form of a heat map. Differences in 2D structure (Δ 2D structure) are highlighted in grey.

Using the miRarchitect tool, artificial miRNAs according to the present invention were designed to target the TMPRSS-2 transcript (GenBank accession number: NM_005656, transcript: ENST00000332149). The amiRNAs were designed on three scaffolds/carriers: pri-miRNA-122, pri-miR-135b, and pri-miR-155. From the candidate ranking list, 8 amiRNAs were selected: 3 based on the pri-miRNA-122 scaffold, 3 on the pri-miRNA-135b scaffold, and 2 on the pri-miRNA-155 scaffold for testing in HEK293T cells (described in Example 1, FIG. 1).

Production of the amiRNA based on the amiRNA candidate selected in step (5) was achieved by chemical synthesis of the amiRNA precursor along with the flanking sequence promoting proper maturation, followed by cloning of this sequence into a target plasmid enabling amiRNA expression in eukaryotic cells. The amiRNA sequences according to the present invention were synthesized by GeneScript and cloned into the pcDNA3.1/Hygro(+) (Thermo Fisher Scientific) at the HindIII and EcoRI restriction sites downstream of the CMV promoter. 4 µg of each plasmid was used to transform *E. coli* TOP10 cells, followed by amplification and isolation of the plasmid. This was a standard procedure for the synthesis, cloning, and subsequent purification of plasmids known to specialists in the field.

The subject of the present invention is also an expression cassette that contains a regulated or constitutive promoter functionally linked to a nucleic acid coding sequence (artificial miRNA) as defined above.

The term "functionally linked" means that the promoter is linked in a manner that allows it to function with the nucleic acid sequences according to the present invention, enabling transcription of the nucleic acid (expression of genetic information, formation of the miRNA molecule) upon introduction into a cell.

According to the present invention, the promoter is a Pol II RNA promoter. In another embodiment, the promoter is a Pol III RNA promoter. According to the present invention, the Pol II RNA promoter is the CMV promoter (human cytomegalovirus immediate early enhancer/promoter). In another embodiment, the Pol II RNA promoter is the CAG promoter (a hybrid construct consisting of a cytomegalovirus (CMV) enhancer fused to a modified chicken β-actin promoter).

An expression cassette according to the present invention may further comprise a polyadenylation signal. In one embodiment, the polyadenylation signal is hGH poly A (growth-hormone polyadenylation signal). In another embodiment, the polyadenylation signal is SV40L poly A (Simian virus 40 late polyadenylation signal), and in yet another embodiment, the polyadenylation signal is short polyA. In one embodiment, the expression cassette according to the present invention further comprises WPRE (Woodchuck hepatitis virus posttranscriptional regulatory element). Such expression cassettes are produced by methods known in the art.

The present invention also encompasses an expression vector containing a nucleic acid molecule as defined above or an expression cassette as defined above. The expression vector of the present invention is a vector selected from an adeno-associated virus (AAV), a lentivirus, an adenovirus, a plasmid, an anellavirus, a non-viral DNA vector, a minimal DNA vector, and a non-integrating lentivirus. Such vectors are produced by methods known in the art.

In a preferred embodiment, the vector is an adeno-associated virus (AAV), which has numerous advantages, including the absence of integration into the host genome, long-term expression of the transgene, and the absence of toxicity.

The present invention also relates to a pharmaceutical composition for silencing the expression of the gene encoding the TMPRSS-2 protein, comprising at least one nucleic acid molecule according to the present invention, or an expression cassette as defined above, or a vector as defined above, and a pharmaceutically acceptable carrier. The pharmaceutically acceptable carrier is selected from a stabilizing buffer, a protective protein, a stability-enhancing agent, and a means for *in vivo* administration. Stabilizing buffers (base carrier) suitable for use as a pharmaceutically acceptable carrier in the pharmaceutical composition of the present invention include:
- Phosphate-buffered saline (PBS) - frequently used as a base solution.
- Tris-HCl, HEPES - pH regulation.
- NaCl, MgCl₂, KCl - ensure ionic balance.
- Protective sugars: e.g., sucrose, trehalose - protect against denaturation during storage or lyophilization.

Protective proteins that can be used as a pharmaceutically acceptable carrier in a pharmaceutical composition according to the present invention include human serum albumin (HSA)-often added to formulations, it protects viruses from adsorption to surfaces.
Stability-enhancing substances that can be used as a pharmaceutically acceptable carrier in a pharmaceutical composition according to the present invention include:
- Polysorbates (e.g., Polysorbate 80)-surfactants that prevent virus aggregation.
- PEG (polyethylene glycol)-used to concentrate viruses, but may also be part of the formulation.

Formulations for *in vivo* administration that can be used as a pharmaceutically acceptable carrier in a pharmaceutical composition according to the present invention include:
- Lipids or nanoparticles.
- Hydrogels, microspheres - for controlled release in tissue.

Such pharmaceutical compositions are prepared by standard methods known to those skilled in the art.

The composition of the invention may be administered by any route of administration, for example, enterally, such as orally, or parenterally, such as by intravenous, intramuscular, or subcutaneous injection, etc. It may also be administered intranasally or by inhalation. The composition of the present invention is advantageously in the form of a spray. The composition of the present invention is advantageously distributed in the respiratory system, in cells and tissues exposed to contact with viruses. According to the present invention, lowering the level of TMPRSS-2 protein in these cells limits the ability of TMPRSS-2-dependent viruses to enter cells, thereby limiting the development of infection.

The artificial miRNAs of the present invention inhibit TMPRSS-2 expression, thereby reducing TMPRSS-2 protein levels, and consequently inhibit viral entry into host cells. The inventors of this invention note that their use in humans may constitute an effective preventive measure against viral infections in high-risk groups and achieve a therapeutic effect in patients affected by TMPRSS-2-dependent viral diseases.

Furthermore, the nucleic acid molecules designed and produced by the inventors of this invention, by modulating protein expression regulation, can be used as a component in therapy, particularly in antiviral therapy.

The nucleic acid molecules of the present invention may be used in the treatment and/or prevention of a viral disease dependent on type 2 transmembrane serine protease selected from SARS, MERS, COVID-19, influenza, parainfluenza, respiratory syncytial virus (RSV) infection, and Zika fever.

The solutions according to the present invention allow for the silencing of the gene encoding the TMPRSS-2 protein. The present invention may therefore be used to silence the expression and regulate the TMPRSS-2 protein (FIGS. 2, 3, 4, and 6) by using an RNAi method based on microRNA.

### EXAMPLE 1

### amiRNAs effectively silence the expression of the TMPRSS-2 gene at the transcript and protein levels

Analysis of TMPRSS-2 transcript and protein levels was performed in HEK293T cells transfected with plasmids for the expression of TMPRSS-2v1 and amiRNA.

### Silencing of the luciferase reporter by anti-TMPRSS-2 amiRNA according to the present invention

With reference to FIG. 2, the results of an *in vitro* analysis of the efficacy of the anti-TMPRSS-2 amiRNA, which is the subject of the invention, are presented. The graph shown in FIG. 2 illustrates the results of luciferase reporter silencing by the anti-TMPRSS-2 amiRNA according to the present invention. HEK293T cells (human embryonic kidney, a cell line derived from human fetal kidney) were transfected with a plasmid encoding TMPRSS-2 and the Luc reporter protein (psiCHECK-2_TMPRSS-2v.1) as well as vectors encoding individual amiRNAs (pcDNA3.1_amiRNA) or control plasmids (pcDNA3.1_pri-miRNA). The efficiency of TMPRSS-2 silencing by amiRNA is presented as the ratio of Renilla/firefly luciferase levels normalized to the corresponding control. The bar chart shows the mean normalized ratio of Renilla luciferase activity to firefly luciferase activity, reflecting the relative level of the TMPRSS2 transcript ± SEM, obtained from at least three independent biological replicates and three technical replicates, 24 hours after cell transfection. The graph also indicates the statistical significance of the observed changes. Detailed data are also presented in the table in FIG. 6.

Cells were cultured in DMEM (Dulbecco's Modified Eagle's Medium, Gibco) supplemented with 10% fetal bovine serum (FBS, BioWest), PenStrep (Gibco), and L-glutamine (Gibco). Cells were cultured at 37°C in 5% CO₂ in humidified air. The plasmid for expressing the target sequence-psiCHEK2_TMPRSS-2v1-encoded two reporter genes (Renilla and firefly luciferases) under the SV40 and HSV-TK promoters, respectively (Promega). For amiRNA design, the natural precursors of human pri-miRNA-122, pri-miRNA-135b, and pri-miRNA-155 were selected, which, according to the mirGeneDB database (Clarke et al., 2025), are sources of homogeneous products arising from the 5' end. The proprietary miRarchitect tool was used to design the amiRNAs; this tool employs machine learning to optimize the sequence and structure of the resulting amiRNAs in order to make them resemble natural miRNA precursors. The amiRNA sequences were synthesized (GeneScript) and cloned into the pcDNA3.1/Hygro(+) plasmid (Thermo Fisher Scientific) under the CMV promoter. HEK293T cells were transfected in a 96-well plate with the psiCHECK-2_TMPRSS-2v.1 and pcDNA3.1_amiRNA plasmids in a 1:10 ratio using Lipofectamine 2000 (Invitrogen, Thermo Fisher Scientific, Carlsbad, CA). The pcDNA3.1_pri-miRNA plasmid, encoding natural pri-miRNA, was used as a control. After 24 hours, the cells were harvested and lysed using Dual-Glo^{®} Luciferase Assay Reagent (Promega, Madison, WI, USA). The bioluminescence assay was performed using the Dual-Glo^{®} Luciferase Assay protocol (Promega) and a Victor Nivo Plate Reader (PerkinElmer, Waltham, MA) in accordance with the manufacturer's instructions. The extent of TMPRSS-2 expression silencing by amiRNA was determined based on the ratio of Renilla luciferase levels to firefly luciferase levels normalized to the control.

Result: The most effective amiRNAs (amiRNA_122_02, amiRNA_135b_01, and amiRNA_135b_02) reduced TMPRSS-2 levels using a luciferase reporter system by approximately 80% (FIG. 2).

### The activity of the amiRNAs was also determined at the transcript and protein levels in a different experimental setup.

HEK293T cells (9×10⁵ cells) were seeded onto a 3.5-cm-diameter plate, and after 24 hours, the cells were transfected with 4 µg of the pcDNA3.1_amiRNA plasmid using 6 µl of Lipofectamine 2000. After another 2 hours, the cells were transfected with 2 µg of the plasmid encoding the target gene (pcDNA3.1_TMPRSS-2/V5). After 48 hours, the cells were harvested, and total RNA (Total RNA Mini Plus D, AA Biotechnology) and protein were isolated. The level of the TMPRSS-2 transcript was determined by RT-qPCR using the CFX Connect Real-Time PCR Detection System (Bio-Rad, Hercules, CA) and the iTaqTM Universal SYBR^{®} Green One-Step Kit (Bio-Rad). β-actin and GAPDH were used as reference genes. Protein was isolated using RIPA buffer (Thermo Scientific). 20 µg of total protein was mixed with 4x Laemmli buffer (Bio-Rad) containing β-mercaptoethanol and heated at 95°C for 5 min. The sample was separated on a 4-15% SDS polyacrylamide gel using Mini PROTEAN^{®} TGX^{™} (Tris-Glycine-eXtended) Precast Gels (Bio-Rad). Electrophoresis was performed in Tris/Glycine/SDS buffer (Bio-Rad) at 150 V. The proteins were then transferred to a nitrocellulose membrane (Amersham Protran 0.45 NC, Cytivia) using a wet transfer method in Tris/Glycine/methanol buffer, at 4°C, 100 V, for 1 h. The membrane was blocked with 10% milk in PBS/0.1% Tween 20 buffer. Primary antibodies used were anti-V5 Tag (ThermoFisher, catalog no. R961-25) and anti-GAPDH (ThermoFisher, cat. no. MA1-16757) and a secondary goat anti-mouse IgG (H+L) antibody conjugated with HRP (ThermoFisher, cat. no. 31430). Immunological assays were performed using the Westar Supernova (XLS3,0100, Cyagen). GAPDH was used as a reference protein.

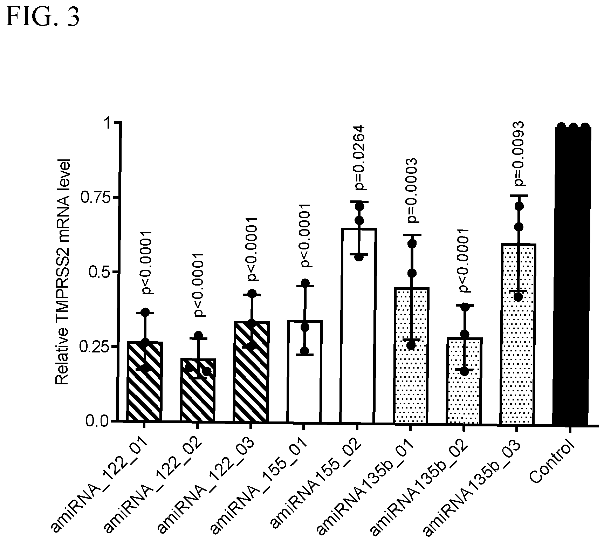

Referring to FIG. 3, the results of the analysis of TMPRSS-2 transcript levels following the application of the amiRNA inhibitors, which are the subject of the invention, are presented. HEK293T cells were transfected with a plasmid encoding TMPRSS-2 (pcDNA3.1/V5_TMPRSS2v1). At the same time, plasmids encoding amiRNA (pCDH_amiRNA) or control plasmids (pCDH_pri-miRNA) were introduced into the cells. The level of the TMPRSS-2 transcript was measured by RT-qPCR and normalized relative to GAPDH and actin mRNA levels. The bar chart shows the mean level of the TMPRSS-2 transcript relative to GAPDH and actin ± SEM (from at least three biological and three technical replicates) 24 hours after cell transfection. The graph also indicates the statistical significance of the observed changes; ns indicates that p > 0.05. Detailed data are also presented in the table in FIG. 6.

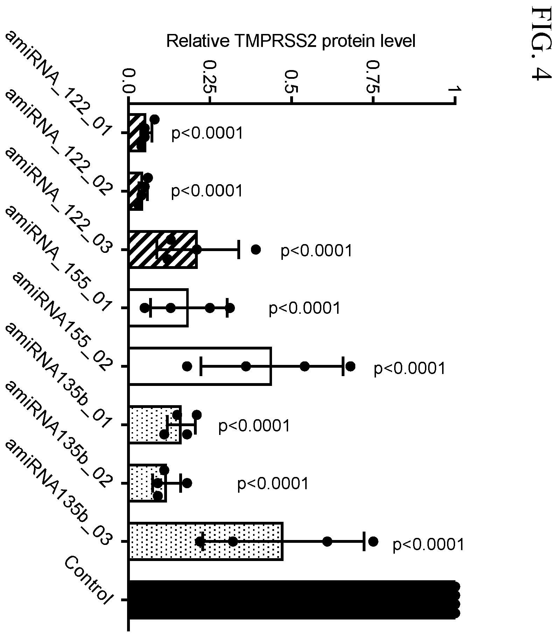

Referring to FIG. 4, the results of the analysis of TMPRSS-2 protein levels following the use of the amiRNA inhibitors described in the invention are presented. HEK293T cells were transfected with a plasmid encoding TMPRSS-2 (pcDNA3.1/V5_TMPRSS2v1) and with pCDH_amiRNA plasmids encoding individual amiRNAs or pCDH_pri-miRNA plasmids encoding natural pri-miRNAs (control). The analysis was performed 24 hours after plasmid administration using the Western blot method. TMPRSS-2 protein levels were normalized relative to GAPDH protein levels. The bars represent the relative mean TMPRSS-2 protein levels, ± SEM (from four biological and three technical replicates). The relative ratio of TMPRSS-2 protein levels after treating cells with individual amiRNAs was compared to the corresponding controls (natural pri-miRNAs) using one-way analysis of variance, multiple comparisons, and Tukey's multiple comparison test. P-values are indicated on the graphs; ns indicates that the p-value is >0.05. Detailed data are also presented in the table in FIG. 6.

Referring to FIG. 6, a summary of the results of the analysis of the activity of anti-TMPRSS-2 amiRNA according to the present invention in HEK293T cells is presented. The table shows the mean values of the luciferase reporter, TMPRSS-2 transcript and protein levels, and standard deviations. Statistical significance is shown in FIG. 2, 3, and 4.

Referring to FIG. 7, the level of the sense strand of amiRNA_122_02 according to the present invention in HEK293T cells is shown in comparison to the level of endogenous miRNAs. The analysis was performed based on the measurement of read counts in small RNA sequencing following transfection of cells with the pcDNA3.1_amiRNA_122_02 expression plasmid. The level of the amiRNA_122_02 guide strand released represents 0.546% of the level of the most abundant endogenous miRNAs in the cell (Top 50).

Result: The most significant reduction in TMPRSS-2 transcript and protein levels, approximately 80% and 95%, respectively, was observed in cells treated with amiRNA_122_02 (FIG. 3 and FIG. 4). Six of the eight amiRNAs reduced TMPRSS-2 protein levels by ≥ 75% (FIG. 4). The most effective amiRNA_122_02 is highly specific-it releases a homogeneous guide strand from the 5' arm of the precursor (FIG. 5), its cellular abundance is low, resembling the level of endogenous miRNAs (FIG. 7), and it has no complementary sequences in the human genome (no risk of off-target effects). These characteristics were achieved through the use of a proprietary amiRNA design method utilizing the miRarchitect tool.

The above description of the embodiments is provided to enable a person skilled in the art to make or use the present invention. Various modifications of the embodiments described are also possible, including all such changes, modifications, and variations, that fall within the spirit and scope of the appended patent claims. The fundamental principles set forth herein may therefore be applied to other embodiments without departing from the scope of the invention. Thus, the intent of the present invention is not to limit it to the embodiments presented herein, but to conform to the broadest scope consistent with the principles and novel features presented herein.

### APPLICATION OF THE INVENTION

The invention relates to the silencing of gene expression using RNA interference (RNAi) technology reagents, particularly artificial microRNAs for use in humans in the treatment and/or prevention of TMPRSS-2-dependent viral diseases. The reagents are nucleic acid molecules according to the present invention, constituting artificial miRNAs (amiRNAs) targeted at the TMPRSS-2 transcript, thereby reducing the levels of the TMPRSS-2 transcript and protein, and consequently capable of inhibiting viral entry into cells.

Their use in humans may serve as an effective preventive measure against viral infections in high-risk groups (e.g., healthcare workers, chronically ill patients, the elderly, post-chemotherapy patients, immunocompromised individuals, those with autoimmune diseases, etc.) and achieve a therapeutic effect in patients affected by TMPRSS-2-dependent viral diseases. Their use in humans may serve as an effective preventive measure against viral infections in high-risk groups (e.g., healthcare workers, patients with chronic illnesses, the elderly, post-chemotherapy patients, immunocompromised individuals, those with autoimmune diseases, etc.) and achieve a therapeutic effect in patients affected by TMPRSS-2-dependent viral diseases.

### REFERENCES

Clarke, A. W., Høye, E., Hembrom, A. A., Paynter, V. M., Vinther, J., Wyrożemski, 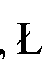 ., Biryukova, I., Formaggioni, A., Ovchinnikov, V., Herlyn, H., Pierce, A., Wu, C., Aslanzadeh, M., Cheneby, J., Martinez, P., Friedländer, M. R., Hovig, E., Hackenberg, M., Umu, S. U., ... Fromm, B. (2025). MirGeneDB 3.0: Improved taxonomic sampling, uniform nomenclature of novel conserved microRNA families, and updated covariance models. *Nucleic Acids Research*, *53* (D1), D116-D128. https://doi.org/10.1093/nar/gkae1094
Galka-Marciniak, P., Olejniczak, M., Starega-Roslan, J., Szczesniak, M. W., Makalowska, I., & Krzyzosiak, W. J. (2016). siRNA release from pri-miRNA scaffolds is controlled by the sequence and structure of RNA. *Biochimica Et Biophysica Acta, 1859* (4), 639-649. https://doi.org/10.1016/j.bbagrm.2016.02.014
Gao, Y.-F., Yu, L., Wei, W., Li, J.-B., Luo, Q.-L., & Shen, J.-L. (2008). Inhibition of hepatitis B virus gene expression and replication by artificial microRNA. World Journal of Gastroenterology, 14 (29), 4684-4689. https://doi.org/10.3748/wjg.14.4684
Guda, S., Brendel, C., Renella, R., Du, P., Bauer, D. E., Canver, M. C., Grenier, J. K., Grimson, A. W., Kamran, S. C., Thornton, J., De Boer, H., Root, D. E., Milsom, M. D., Orkin, S. H., Gregory, R. I., & Williams, D. A. (2015). miRNA-embedded shRNAs for Lineage-specific BCL11A Knockdown and Hemoglobin F Induction. Molecular Therapy, 23 (9), 1465-1474. https://doi.org/10.1038/mt.2015.113
Gunst, J. D., Staerke, N. B., Pahus, M. H., Kristensen, L. H., Bodilsen, J., Lohse, N., Dalgaard, L. S., Brønnum, D., Fröbert, O., Hønge, B., Johansen, I. S., Monrad, I., Erikstrup, C., Rosendal, R., Vilstrup, E., Mariager, T., Bove, D. G., Offersen, R., Shakar, S., ... Søgaard, O. S. (2021). Efficacy of the TMPRSS2 inhibitor camostat mesilate in patients hospitalized with Covid-19-a double-blind randomized controlled trial. EClinicalMedicine, 35 , 100849. https://doi.org/10.1016/j.eclinm.2021.100849
Karothia, D., Kumar Dash, P., Parida, M., Bhagyawant, S. S., & Kumar, J. S. (2020). Vector-derived artificial miRNA-mediated inhibition of West Nile virus replication and protein expression. Gene, 729 , 144300. https://doi.org/10.1016/j.gene.2019.144300
Kim, T. S., Heinlein, C., Hackman, R. C., & Nelson, P. S. (2006). Phenotypic analysis of mice lacking the Tmprss2-encoded protease. Molecular and Cellular Biology, 26 (3), 965-975. https://doi.org/10.1128/MCB.26.3.965-975.2006
Kim, Y., Bae, S., Chang, H.-H., & Kim, S.-W. (2023). Long COVID prevalence and impact on quality of life 2 years after acute COVID-19. Scientific Reports, 13 (1), 11207. https://doi.org/10.1038/s41598-023-36995-4
Kotowska-Zimmer, A., Pewinska, M., & Olejniczak, M. (2021). Artificial miRNAs as therapeutic tools: Challenges and opportunities. WIREs RNA, 12 (4), e1640. https://doi.org/10.1002/wrna.1640
Mantzourani, C., Vasilakaki, S., Gerogianni, V.-E., & Kokotos, G. (2022). The discovery and development of transmembrane serine protease 2 (TMPRSS2) inhibitors as candidate drugs for the treatment of COVID-19. Expert Opinion on Drug Discovery, 17 (3), 231-246. https://doi.org/10.1080/17460441.2022.2029843
Mantzourani, C., Vasilakaki, Sofia, Gerogianni ,Velisaria-Eleni, & Kokotos, G. (2022). The discovery and development of transmembrane serine protease 2 (TMPRSS2) inhibitors as candidate drugs for the treatment of COVID-19. Expert Opinion on Drug Discovery, 17 (3), 231-246. https://doi.org/10.1080/17460441.2022.2029843
Miniarikova, J., Zanella, I., Huseinovic, A., van der Zon, T., Hanemaaijer, E., Martier, R., Koornneef, A., Southwell, A. L., Hayden, M. R., van Deventer, S. J., Petry, H., & Konstantinova, P. (2016). Design, Characterization, and Lead Selection of Therapeutic miRNAs Targeting Huntingtin for Development of Gene Therapy for Huntington's Disease. Molecular Therapy. Nucleic Acids, 5 (3), e297. https://doi.org/10.1038/mtna.2016.7
Mohan, E. C., Savarraj, J. P. J., Colpo, G. D., Morales, D., Finger, C. E., McAlister, A., Ahnstedt, H., Choi, H., McCullough, L. D., & Manwani, B. (2024). Aromatase, testosterone, TMPRSS2: Determinants of COVID-19 severity. Biology of Sex Differences, 15 (1), 84. https://doi.org/10.1186/s13293-024-00658-4
Muhuri, M., Levy, D. I., Schulz, M., McCarty, D., & Gao, G. (2022). Durability of transgene expression after rAAV gene therapy. Molecular Therapy, 30 (4), 1364-1380. https://doi.org/10.1016/j.ymthe.2022.03.004
Nimishakavi, S., Raymond, W. W., Gruenert, D. C., & Caughey, G. H. (2015). Divergent Inhibitor Susceptibility among Airway Lumen-Accessible Tryptic Proteases. PLOS ONE, 10 (10), e0141169. https://doi.org/10.1371/journal.pone.0141169
Nowak, R., Gazecka, M., Hoffmann, M., Kierzek, R., Pöhlmann, S., & Zmora, P. (2024). TMPRSS2-specific antisense oligonucleotides inhibit host cell entry of emerging viruses. Virology, 600, 110218. https://doi.org/10.1016/j.virol.2024.110218
Olejniczak, M., Galka, P., & Krzyzosiak, W. J. (2010). Sequence-non-specific effects of RNA interference triggers and microRNA regulators. Nucleic Acids Research, 38 (1), 1-16. https://doi.org/10.1093/nar/gkp829
Park, S., Park, S.-H., Oh, J.-S., Noh, Y.-K., Hur, J. K., & Nam, J.-W. (2024). shRNAI: A deep neural network for the design of highly potent shRNAs (p. 2024.01.09.574789). bioRxiv. https://doi.org/10.1101/2024.01.09.574789
Pelossof, R., Fairchild, L., Huang, C.-H., Widmer, C., Sreedharan, V. T., Sinha, N., Lai, D.-Y., Guan, Y., Premsrirut, P. K., Tschaharganeh, D. F., Hoffmann, T., Thapar, V., Xiang, Q., Garippa, R. J., Rätsch, G., Zuber, J., Lowe, S. W., Leslie, C. S., & Fellmann, C. (2017). Prediction of potent shRNAs with a sequential classification algorithm. Nature Biotechnology, 35 (4), 350-353. https://doi.org/10.1038/nbt.3807
Redondo-Calvo, F. J., Padin, J. F., Martínez-Alarcón, J., Muñoz-Rodríguez, J. R., Serrano-Oviedo, L., López-Juárez, P., Porras Leal, M. L., González Gasca, F. J., Rodriguez Martinez, M., Pérez Serrano, R., Sánchez Cadena, A., Bejarano-Ramírez, N., Muñoz Hornero, C., Villegas Fernández-Infantes, M. D., Manrique Romo, M. I., Gómez-Romero, F. J., Pérez-Ortiz, J. M., & Team, A. (2022). Inhaled aprotinin reduces viral load in mild-to-moderate inpatients with SARS-CoV-2 infection. European Journal of Clinical Investigation, 52 (11), e13850. https://doi.org/10.1111/eci.13850
Sharma, H., Tripathi, A., Kumari, B., Vrati, S., & Banerjee, A. (2018). Artificial MicroRNA-Mediated Inhibition of Japanese Encephalitis Virus Replication in Neuronal Cells. Nucleic Acid Therapeutics, 28 (6), 357-365. https://doi.org/10.1089/nat.2018.0743
Tang, Y., Ebadi, M., Lei, J., Feng, Z., Fakhari, S., Wu, P., Smith, M. D., Limberis, M. P., Kolbeck, R., Excoffon, K. J., Yan, Z., & Engelhardt, J. F. (2024). Durable transgene expression and efficient re-administration after rAAV2.5T-mediated fCFTRΔR gene delivery to adult ferret lungs. Molecular Therapy - Methods & Clinical Development, 32 (2), 101244. https://doi.org/10.1016/j.omtm.2024.101244
Xie, P., Xie, Y., Zhang, X., Huang, H., He, L., Wang, X., & Wang, S. (2013). Inhibition of Dengue Virus 2 Replication by Artificial MicroRNAs Targeting the Conserved Regions. Nucleic Acid Therapeutics, 23 (4), 244-252. https://doi.org/10.1089/nat.2012.0405
Zhang, T., Cheng, T., Wei, L., Cai, Y., Yeo, A. E., Han, J., Yuan, Y. A., Zhang, J., & Xia, N. (2012). Efficient inhibition of HIV-1 replication by an artificial polycistronic miRNA construct. Virology Journal, 9 (1), 118. https://doi.org/10.1186/1743-422X-9-118

**Table 1. List of sequences**

| SEQ. ID No. | Identification | 5'-3' sequence, guide strand in =bold and grayed out, passenger strand in =bold and underlined |
|---|---|---|
| 1 | amiRNA_122_01 according to the invention | CGUGGCUACAGAGUUUCCUUAGCAG AGCUGUUCAUAGACAUAUCUGCUG UUGUGUCUAAACUAUCAACAGCAG AUCUGUCUAUUUACAGCUACUGCUA GGCAAUCCUUCCCUCGAUAA |
| 2 | guide strand in amiRNA_122_01 according to the invention | **UUCAUAGACAUAUCUGCUGUUG** |
| 3 | mRNA sequence within the TMPRSS-2 transcript | CAACAGCAGA UAUGUCUAUGAA |
| | that is complementary to the SEQ ID NO. 2 | |
| 4 | amiRNA_122_02 according to the invention | CGUGGCUACAGAGUUUCCUUAGCAG AGCUGUUUAUAGAUAUCGACAUUG CCGUGUCUAAACUAUCAGCAAUGU CGCUAUCUAUGCACAGCUACUGCU AGGCAAUCCUUCCCUCGAUAA |
| 5 | guide strand in amiRNA_122_02 according to the invention, in amiRNA_155_01 according to the invention or in amiRNA_135b_02 according to the invention | **UUUAUAGAUAUCGACAUUGCCG** |
| 6 | mRNA sequence within the TMPRSS-2 transcript that is complementary to the SEQ ID NO. 5 | CGGCAAUGUCGAUAUCUAUAAA |
| 7 | amiRNA_122_03 according to the invention | CGUGGCUACAGAGUUUCCUUAGCAG AGCUGUACUAUUCCUUGGCUAGAG UAAUGUCUAAACUAUCAACUCUAGC CUAGGAAUAACACAGCUACUGCUA GGCAAUCCUUCCCUCGAUAA |
| 8 | guide strand in amiRNA_122_03 according to the invention | **UACUAUUCCUUGGCUAGAGUAA** |
| 9 | mRNA sequence within the TMPRSS-2 transcript that is complementary to the SEQ ID NO. 8 | UUACUCUAGCCAAGGAAUAGUA |
| 10 | amiRNA_155_01 according to the invention | UGCUGAAGGCUUGCUGUAGGCUGUA UGCUGUUUAUAGAUAUCGACAUUG CCGUUUUUGCCUCCAACUGACGGCA AGUCAUAUCUAUAAACAGUGUAUG AUGCCUGUUACUAGCAUUCACAU |
| 11 | amiRNA_155_02 according to the invention | UGCUGAAGGCUUGCUGUAGGCUGUA UGCUGUUUAUAGAUAUCGACAUUG CCGUUUUUGCCUCCAACUGACGGCA AGUCAUAUCUAUAAACAGUGUAUG AUGCCUGUUACUAGCAUUCACAU UGCUGAAGGCUUGCUGUAGGCUGUA UGCUGUCUCAAAAUCCCCGCAAAU GCCUUUUUGCCUCCAACUGAGGCAU UGCGGGAUUUUGAGACAGUGUAUG AUGCCUGUUACUAGCAUUCACAU |
| 12 | guide strand in amiRNA_155_02 according to the invention | **UCUCAAAAUCCCCGCAAAUGCC** |
| 13 | mRNA sequence within the TMPRSS-2 transcript that is complementary to the SEQ ID NO. 12 | GGCAUUUGCGGGGAUUUUGAGA |
| 14 | amiRNA_135b_01 according to the invention | GCCGCUGGACCCCUCCACUCUGCUG UGGCCUUCAGUUUCAUAAAGCUGG UGGAUUGCUGUCCCAAACUCCACCA GCUAUUGAAACUGAAGGCUACAGU GAGGGGCGAGCUCCUUCUCCUGC |
| 15 | guide strand in amiRNA_135b_01 according to the invention | **UUCAGUUUCAUAAAGCUGGUGG** |
| 16 | mRNA sequence within the TMPRSS-2 transcript that is complementary to the SEQ ID NO. 15 | CCACCAGCUUUAUGAAACUGAA |
| 17 | amiRNA_135b_02 according to the invention | GCCGCUGGACCCCUCCACUCUGCUG UGGCCUUUAUAGAUAUCGACAUUG CCGAUUGCUGUCCCAAACUCGGCAA UGUGUUAUCUAUAAAGGCUACAGU GAGGGGCGAGCUCCUUCUCCUGC |
| 18 | amiRNA_135b_03 according to the invention | GCCGCUGGACCCCUCCACUCUGCUG UGGCCUUUAUAGAUAUCGACAUUG CCGAUUGCUGUCCCAAACUCGGCAA UGUGUUAUCUAUAAAGGCUACAGU GAGGGGCGAGCUCCUUCUCCUGC GCCGCUGGACCCCUCCACUCUGCUG UGGCCUCCAAUAGCUGGUGGUGAC CCUAUUGCUGUCCCAAACUCGGGUC ACCUAAGCUAUUGGAGGCUACAGU GAGGGGCGAGCUCCUUCUCCUGC |
| 19 | guide strand in amiRNA_135b_03 according to the invention | **UCCAAUAGCUGGUGGUGACCCU** |
| 20 | mRNA sequence within the TMPRSS-2 transcript that is complementary to the SEQ ID NO. 19 | AGGGUCACCACCAGCUAUUGGA |
| 21 | loop in amiRNA_122_01 according to the invention, amiRNA_122_02 according to the invention or in amiRNA_122_03 according to the invention | UGUCUAAACUAUCA |
| 22 | loop in amiRNA_155_01 according to the invention or in amiRNA_155_02 according to the invention | UUUUUGCCUCCAACUGA |
| 23 | loop in amiRNA_135b_01 according to the invention, amiRNA_135b_02 according to the invention or in amiRNA_135b_03 | AUUGCUGUCCCAAACUC |
| 24 | passenger strand in amiRNA_122_01 according to the invention | **ACAGCAGAUCUGUCUAUUUACA** |
| 25 | passenger strand in amiRNA_122_02 according to the invention | **GCAAUGUCGCUAUCUAUGCACA** |
| 26 | passenger strand in amiRNA_122_03 according to the invention | **ACUCUAGCCUAGGAAUAACACA** |
| 27 | passenger strand in amiRNA_155_02 according to the invention | **GGCAUUGCGGGAUUUUGAGACA** |
| 28 | passenger strand in amiRNA_135b_01 according to the invention | **CACCAGCUAUUGAAACUGAAGG** |
| 29 | passenger strand in amiRNA_135b_03 according to the invention | **GGGUCACCUAAGCUAUUGGAGG** |
| 30 | passenger strand in amiRNA_155_01 according to the invention | **CGGCAAGUCAUAUCUAUAAACA** |
| 31 | passenger strand in amiRNA_135b_02 according to the invention | **GGCAAUGUGUUAUCUAUAAAGG** |

List of sequences in a version in accordance with
the WIPO ST.25 standard

<110> Instytut Chemii Bioorganicznej Polskiej Akademii Nauk

<120> A nucleic acid molecule for silencing the expression of the gene encoding
the TMPRSS-2 protein, expression cassette, expression vector, pharmaceutical
composition, nucleic acid molecule for therapeutic use, nucleic acid molecule
for use in the treatment and/or prevention of a TMPRSS-2-dependent viral
disease, and an in vitro method for silencing the expression of the gene encoding
the TMPRSS-2 protein

<130> 14P55097PL00

<160> 29

<170> BiSSAP 1.3.6

<210> 1
<211> 118
<212> RNA
<213> Artificial sequence

<400> 1
cguggcuaca gaguuuccuu agcagagcug uucauagaca uaucugcugu ugugucuaaa     60

cuaucaacag cagaucuguc uauuuacagc uacugcuagg caauccuucc cucgauaa      118

<210> 2
<211> 22
<212> RNA
<213> Artificial sequence

<400> 2
uucauagaca uaucugcugu ug                                              22

<210> 3
<211> 22

<212> RNA
<213> Homo sapiens

<400> 3
caacagcaga uaugucuaug aa                                                22

<210> 4
<211> 118
<212> RNA
<213> Artificial sequence

<400> 4
cguggcuaca gaguuuccuu agcagagcug uuuauagaua ucgacauugc cgugucuaaa      60

cuaucagcaa ugucgcuauc uaugcacagc uacugcuagg caauccuucc cucgauaa       118

<210> 5
<211> 22
<212> RNA
<213> Artificial sequence

<400> 5
uuuauagaua ucgacauugc cg                                                22

<210> 6
<211> 22
<212> RNA
<213> Homo sapiens

<400> 6
cggcaauguc gauaucuaua aa                                                22

<210> 7

<211> 118

<212> RNA

<213> Artificial sequence

<400> 7

cguggcuaca gaguuuccuu agcagagcug uacuauuccu uggcuagagu aaugucuaaa 60

cuaucaacuc uagccuagga auaacacagc uacugcuagg caauccuucc cucgauaa 118

<210> 8

<211> 22

<212> RNA

<213> Artificial sequence

<400> 8

uacuauuccu uggcuagagu aa 22

<210> 9

<211> 22

<212> RNA

<213> Homo sapiens

<400> 9

uuacucuagc caaggaauag ua 22

<210> 10

<211> 121

<212> RNA

<213> Artificial sequence

<400> 10

ugcugaaggc uugcuguagg cuguaugcug uuuauagaua ucgacauugc cguuuuugcc 60

uccaacugac ggcaagucau aucuauaaac aguguaugau gccuguuacu agcauucaca 120

u                                                                   121

<210> 11
<211> 121
<212> RNA
<213> Artificial sequence

<400> 11
ugcugaaggc uugcuguagg cuguaugcug ucucaaaauc cccgcaaaug ccuuuuugcc      60

uccaacugag gcauugcggg auuuugagac aguguaugau gccuguuacu agcauucaca     120

u                                                                   121

<210> 12
<211> 22
<212> RNA
<213> Artificial sequence

<400> 12
ucucaaaauc cccgcaaaug cc                                             22

<210> 13
<211> 22
<212> RNA
<213> Homo sapiens

<400> 13
   ggcauuugcg gggauuuuga ga                                              22

<210> 14
<211> 121
<212> RNA

<213> Artificial sequence

<400> 14
gccgcuggac cccuccacuc ugcuguggcc uucaguuuca uaaagcuggu ggauugcugu     60

cccaaacucc accagcuauu gaaacugaag gcuacaguga ggggcgagcu ccuucuccug    120

c                                                                    121

<210> 15
<211> 22
<212> RNA
<213> Artificial sequence

<400> 15
uucaguuuca uaaagcuggu gg                                              22

<210> 16
<211> 22
<212> RNA
<213> Homo sapiens

<400> 16
ccaccagcuu uaugaaacug aa                                              22

<210> 17
<211> 121
<212> RNA
<213> Artificial sequence

<400> 17
gccgcuggac cccuccacuc ugcuguggcc uuuauagaua ucgacauugc cgauugcugu     60

cccaaacucg gcaauguguu aucuauaaag gcuacaguga ggggcgagcu ccuucuccug    120

c                                                                    121

<210> 18
<211> 121
<212> RNA
<213> Artificial sequence

<400> 18
gccgcuggac cccuccacuc ugcuguggcc uccaauagcu gguggugacc cuauugcugu      60

cccaaacucg ggucaccuaa gcuauuggag gcuacaguga ggggcgagcu ccuucuccug     120

c                                                                    121

<210> 19
<211> 22
<212> RNA
<213> Artificial sequence

<400> 19
uccaauagcu gguggugacc cu                                              22

<210> 20
<211> 22
<212> RNA
<213> Homo sapiens

<400> 20
agggucacca ccagcuauug ga                                              22

<210> 21
<211> 14
<212> RNA

<213> Homo sapiens

<400> 21
ugucuaaacu auca                                                          14

<210> 22
<211> 17
<212> RNA
<213> Homo sapiens

<400> 22
uuuuugccuc caacuga                                                       17

<210> 23
<211> 17
<212> RNA
<213> Homo sapiens

<400> 23
auugcugucc caaacuc                                                       17

<210> 24
<211> 22
<212> RNA
<213> Artificial sequence

<400> 24
acagcagauc ugucuauuua ca                                                 22

<210> 25
<211> 22
<212> RNA
<213> Artificial sequence

<400> 25
gcaaugucgc uaucuaugca ca                                                22

<210> 26
<211> 22
<212> RNA
<213> Artificial sequence

<400> 26
acucuagccu aggaauaaca ca                                                22

<210> 27
<211> 22
<212> RNA
<213> Artificial sequence

<400> 27
ggcauugcgg gauuuugaga ca                                                22

<210> 28
<211> 22
<212> RNA
<213> Artificial sequence

<400> 28
caccagcuau ugaaacugaa gg                                                22

<210> 29
<211> 22
<212> RNA
<213> Artificial sequence

<400> 29
gggucaccua agcuauugga gg                                                22

<210> 30
<211> 22
<212> RNA
<213> Artificial sequence

<400> 30
cggcaaguca uaucuauaaa ca                                                22

<210> 31
<211> 22
<212> RNA
<213> Artificial sequence

<400> 31
ggcaaugugu uaucuauaaa gg                                                22

### List of sequences in a version in accordance with the WIPO ST.26 standard

<?xml version="1.0" encoding="UTF-8"?>
<!DOCTYPE ST26SequenceListing PUBLIC "-//WIPO//DTD Sequence Listing 1.3// EN" "ST26SequenceListing_V1_3.dtd"> <ST26SequenceListing originalFreeTextLanguageCode="en" dtdVersion="V1_3" fileName="14P55097EP00 Sequence listing ST26 ost.xml" softwareName="WIPO Sequence" softwareVersion="2.3.0" productionDate="2026-05-11">
<ApplicantFileReference>14P55097EP00</ApplicantFileReference>
<EarliestPriorityApplicationIdentification> <IPOfficeCode>PL</ IPOfficeCode> <ApplicationNumberText>P452059</ApplicationNumberText>
<FilingDate>2025-05-16</FilingDate> </ EarliestPriorityApplicationIdentification>
<ApplicantName languageCode="pl">Instytut Chemii Bioorganicznej Polskiej Akademii Nauk</ApplicantName> <InventionTitle **languageCode="pl">**A nucleic acid molecule for silencing the expression of the gene encoding the TMPRSS-2 protein, expression cassette, expression vector, pharmaceutical composition, nucleic acid molecule for therapeutic use, nucleic acid molecule for use in the treatment and/or prevention of a TMPRSS-2-dependent viral disease, and an *in vitro* method for silencing the expression of the gene encoding the TMPRSS-2 protein**</InventionTitle>**
<SequenceTotalQuantity>31</SequenceTotalQuantity> <SequenceData sequenceIDNumber="1">
<INSDSeq>
<INSDSeq_length>118</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..118</INSDFeature_location>
<INSDFeature_quals>
<INSDQualifier>
<INSDQualifier_name>mol_type</INSDQualifier_name>
<INSDQualifier_value>other RNA</INSDQualifier_value> </
INSDQualifier>
<INSDQualifier id="q1">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>cgtggctacagagtttccttagcagagctgttcatagacatatctgctgttgtgtc taaactatcaacagcagatctgtctatttacagctactgctaggcaatccttccctcgataa</ INSDSeq_sequence> </INSDSeq>
</SequenceData>
<SequenceData sequenceIDNumber="2">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier> <INSDQualifier id="q24">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>ttcatagacatatctgctgttg</ INSDSeq_sequence> </INSDSeq>
</SequenceData>
<SequenceData sequenceIDNumber="3">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q47">
<INSDQualifier_name>organism</INSDQualifier_name>
<INSDQualifier_value>Homo sapiens</INSDQualifier_value> </ INSDQualifier>
</INSDFeature_quals>
</INSDFeature>
</INSDSeq_feature-table> <INSDSeq_sequence>caacagcagatatgtctatgaa</ INSDSeq_sequence> </INSDSeq>
</SequenceData>
<SequenceData sequenceIDNumber="4">
<INSDSeq>
<INSDSeq_length>118</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..118</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q48">
<INSDQualifier_name>organism</INSDQualifier_name>
<INSDQualifier_value>synthetic construct</INSDQualifier_value>
</INSDQualifier>
</INSDFeature_quals>
</INSDFeature> </INSDSeq_feature-table>
<INSDSeq_sequence>cgtggctacagagtttccttagcagagctgtttatagatatcgacattgccgtgtc taaactatcagcaatgtcgctatctatgcacagctactgctaggcaatccttccctcgataa</
INSDSeq_sequence> </INSDSeq>
</SequenceData>
<SequenceData sequenceIDNumber="5">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier name>mol_type</INSDQualifier name>
<INSDQualifier_value>other RNA</INSDQualifier_value> </ INSDQualifier>
<INSDQualifier id="q49">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>tttatagatatcgacattgccg</ INSDSeq_sequence> </INSDSeq>
</SequenceData>
<SequenceData sequenceIDNumber="6">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q50">
<INSDQualifier_name>organism</INSDQualifier_name>
<INSDQualifier_value>Homo sapiens</INSDQualifier _value> </ INSDQualifier>
</INSDFeature_quals>
</INSDFeature>
</INSDSeq_feature-table> <INSDSeq_sequence>cggcaatgtcgatatctataaa</ INSDSeq_sequence> </INSDSeq>
</SequenceData>
<SequenceData sequenceIDNumber="7">
<INSDSeq> <INSDSeq_length>118</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..118</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q51">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>cgtggctacagagtttccttagcagagctgtactattccttggctagagtaatgtc taaactatcaactctagcctaggaataacacagctactgctaggcaatccttccctcgataa</ INSDSeq_sequence> </INSDSeq>
</SequenceData>
<SequenceData sequenceIDNumber="8">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q52">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>tactattccttggctagagtaa</ INSDSeq_sequence> </INSDSeq>
</SequenceData>
<SequenceData sequenceIDNumber="9">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature> <INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q53">
<INSDQualifier_name>organism</INSDQualifier_name>
<INSDQualifier_value>Homo sapiens</INSDQualifier _value> </ INSDQualifier>
</INSDFeature_quals>
</INSDFeature>
</INSDSeq_feature-table> <INSDSeq_sequence>ttactctagccaaggaatagta</ INSDSeq_sequence> </INSDSeq>
</SequenceData>
<SequenceData sequenceIDNumber="10">
<INSDSeq>
<INSDSeq_length>121</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..121</INSDFeature_location>
<INSDFeature_quals> <INSDQualifier>
<INSDQualifier_name>mol_type</INSDQualifier_name>
<INSDQualifier_value>other RNA</INSDQualifier_value> </ INSDQualifier>
<INSDQualifier id="q54">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tgctgaaggcttgctgtaggctgtatgctgtttatagatatcgacattgccgtttt tgcctccaactgacggcaagtcatatctataaacagtgtatgatgcctgttactagcattcacat</ INSDSeq_sequence> </INSDSeq>
</SequenceData>
<SequenceData sequenceIDNumber="11 ">
<INSDSeq>
<INSDSeq_length>121</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..121</INSDFeature_location>
<INSDFeature_quals>
<INSDQualifier>
<INSDQualifier_name>mol_type</INSDQualifier_name>
<INSDQualifier_value>other RNA</1NSDQualifier_value> </ INSDQualifier>
<INSDQualifier id="q55">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tgctgaaggcttgctgtaggctgtatgctgtctcaaaatccccgcaaatgcctttt tgcctccaactgaggcattgcgggattttgagacagtgtatgatgcctgttactagcattcacat</ INSDSeq_sequence> </INSDSeq>
</SequenceData>
<SequenceData sequenceIDNumber="12">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q56">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>tctcaaaatccccgcaaatgcc</ INSDSeq_sequence> </INSDSeq>
</SequenceData>
<SequenceData sequenceIDNumber="13 ">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q57">
<INSDQualifier_name>organism</INSDQualifier_name>
<INSDQualifier_value>Homo sapiens</INSDQualifier _value> </ INSDQualifier>
</INSDFeature_quals>
</INSDFeature>
</INSDSeq_feature-table> <INSDSeq_sequence>ggcatttgcggggattttgaga</ INSDSeq_sequence> </INSDSeq>
</SequenceData>
<SequenceData sequenceIDNumber="14">
<INSDSeq>
<INSDSeq_length>121</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..121</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q58">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gccgctggacccctccactctgctgtggccttcagtttcataaagctggtggattg ctgtcccaaactccaccagctattgaaactgaaggctacagtgaggggcgagctccttctcctgc</ INSDSeq_sequence> </INSDSeq>
</SequenceData>
<SequenceData sequenceIDNumber="15 ">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q59">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>ttcagtttcataaagctggtgg</ INSDSeq_sequence> </INSDSeq>
</SequenceData>
<SequenceData sequenceIDNumber="16">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q60">
<INSDQualifier_name>organism</INSDQualifier_name>
<INSDQualifier_value>Homo sapiens</INSDQualifier _value> </ INSDQualifier>
</INSDFeature_quals>
</INSDFeature> </INSDSeq_feature-table>
<INSDSeq_sequence>ccaccagctttatgaaactgaa</INSDSeq_sequence></INSDSeq>
</SequenceData>
<SequenceData sequenceIDNumber="17">
<INSDSeq>
<INSDSeq_length>121</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..121</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q61">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gccgctggacccctccactctgctgtggcctttatagatatcgacattgccgattg ctgtcccaaactcggcaatgtgttatctataaaggctacagtgaggggcgagctccttctcctgc</ INSDSeq_sequence> </INSDSeq>
</SequenceData>
<SequenceData sequenceIDNumber="18">
<INSDSeq>
<INSDSeq_length>121</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..121</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q62">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gccgctggacccctccactctgctgtggcctccaatagctggtggtgaccctattg ctgtcccaaactcgggtcacctaagctattggaggctacagtgaggggcgagctccttctcctgc</ INSDSeq_sequence> </INSDSeq>
</SequenceData>
<SequenceData sequenceIDNumber="19">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q63">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>tccaatagctggtggtgaccct</ INSDSeq_sequence> </INSDSeq>
</SequenceData>
<SequenceData sequenceIDNumber="20">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q64">
<INSDQualifier_name>organism</INSDQualifier_name>
<INSDQualifier_value>Homo sapiens</INSDQualifier _value> </ INSDQualifier>
</INSDFeature_quals>
</INSDFeature>
</INSDSeq_feature-table> <INSDSeq_sequence>agggtcaccaccagctattgga</ INSDSeq_sequence> </INSDSeq>
</SequenceData>
<SequenceData sequenceIDNumber="21">
<INSDSeq> <INSDSeq_length>14</INSDSeq_length> <INSDSeq_moltype>RNA</ INSDSeq_moltype> <INSDSeq_division>PAT</INSDSeq_division>
<INSDSeq_feature-table>
<INSDFeature>
<INSDFeature_key>source</INSDFeature_key>
<INSDFeature_location>1..14</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q65">
<INSDQualifier_name>organism</INSDQualifier_name>
<INSDQualifier_value>Homo sapiens</INSDQualifier _value> </ INSDQualifier>
</INSDFeature_quals>
</INSDFeature>
</INSDSeq_feature-table> <INSDSeq_sequence>tgtctaaactatca</ INSDSeq_sequence> </INSDSeq>
</SequenceData>
<SequenceData sequenceIDNumber="22">
<INSDSeq>
<INSDSeq_length>17</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..17</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q66">
<INSDQualifier_name>organism</INSDQualifier_name>
<INSDQualifier_value>Homo sapiens</INSDQualifier _value> </ INSDQualifier>
</INSDFeature_quals>
</INSDFeature>
</INSDSeq_feature-table> <INSDSeq_sequence>tttttgcctccaactga</ INSDSeq_sequence> </INSDSeq>
</SequenceData>
<SequenceData sequenceIDNumber="23">
<INSDSeq>
<INSDSeq_length>17</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..17</INSDFeature_location>
<INSDFeature_quals> <INSDQualifier>
<INSDQualifier_name>mol_type</INSDQualifier_name>
<INSDQualifier_value>other RNA</INSDQualifier_value> </ INSDQualifier>
<INSDQualifier id="q67">
<INSDQualifier_name>organism</INSDQualifier_name>
<INSDQualifier_value>Homo sapiens</INSDQualifier _value> </ INSDQualifier>
</INSDFeature_quals>
</INSDFeature>
</INSDSeq_feature-table> <INSDSeq_sequence>attgctgtcccaaactc</ INSDSeq_sequence> </INSDSeq>
</SequenceData>
<SequenceData sequenceIDNumber="24">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q68">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>acagcagatctgtctatttaca</ INSDSeq_sequence> </INSDSeq>
</SequenceData>
<SequenceData sequenceIDNumber="25">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q69">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>gcaatgtcgctatctatgcaca</
INSDSeq_sequence> </INSDSeq>
</SequenceData>
<SequenceData sequenceIDNumber="26">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q70">
<INSDQualifier_name>organism</INSDQualifier_name>
<INSDQualifier_value>synthetic construct</INSDQualifier_value>
</INSDQualifier>
</INSDFeature_quals>
</INSDFeature> </INSDSeq_feature-table>
<INSDSeq_sequence>actctagcctaggaataacaca</INSDSeq_sequence> </INSDSeq>
</SequenceData>
<SequenceData sequenceIDNumber="27">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q71">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>ggcattgcgggattttgagaca</ INSDSeq_sequence> </INSDSeq>
</SequenceData> <SequenceData sequenceIDNumber="28">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q72">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>caccagctattgaaactgaagg</ INSDSeq_sequence> </INSDSeq>
</SequenceData>
<SequenceData sequenceIDNumber="29">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q73 ">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>gggtcacctaagctattggagg</ INSDSeq_sequence> </INSDSeq>
</SequenceData>
<SequenceData sequenceIDNumber="30">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q76">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>cggcaagtcatatctataaaca</ INSDSeq_sequence> </INSDSeq>
</SequenceData>
<SequenceData sequenceIDNumber="31">
<INSDSeq>
<INSDSeq_length>22</INSDSeq_length> <INSDSeq_moltype>RNA</INSDSeq_moltype>
<INSDSeq_division>PAT</INSDSeq_division> <INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other RNA</INSDQualifier_value> </ INSDQualifier>
<INSDQualifier id="q78">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table> <INSDSeq_sequence>ggcaatgtgttatctataaagg</ INSDSeq_sequence> </INSDSeq>
</SequenceData>
</ST26SequenceListing>

Sequence Listing Information:
   DTD Version: V1_3
   File Name: 14P55097PL00 Sequence listing ST26.xml
   Software Name: WIPO Sequence
   Software Version: 2.3.0
   Production Date: 2025-05-15
General Information:
   Current application / Applicant file reference: 14P55097PL00
   Applicant name: Instytut Chemii Bioorganicznej Polskiej Akademii Nauk
   Applicant name / Language: pl
   Invention title: A nucleic acid molecule for silencing the expression of the gene encoding the TMPRSS-2 protein, expression cassette, expression vector, pharmaceutical composition, nucleic acid molecule for therapeutic use, nucleic acid molecule for use in the treatment and/or prevention of a TMPRSS-2-dependent viral disease, and an *in vitro* method for silencing the expression of the gene encoding the TMPRSS-2 protein ( eng )
   Sequence Total Quantity: 29
Sequences:
   Sequence Number (ID): 1
   Length: 118
   Molecule Type: RNA
   Features Location/Qualifiers:
      - source, 1..118
         > mol_type, other RNA
         > organism, synthetic construct
   Residues:
      cgtggctaca gagtttcctt agcagagctg ttcatagaca tatctgctgt tgtgtctaaa 60 ctatcaacag cagatctgtc tatttacagc tactgctagg caatccttcc ctcgataa 118
   Sequence Number (ID): 2
   Length: 22
   Molecule Type: RNA
   Features Location/Qualifiers:
      - source, 1..22
         > mol_type, other RNA
         > organism, synthetic construct
   Residues:
      ttcatagaca tatctgctgt tg 22
   Sequence Number (ID): 3
   Length: 22
   Molecule Type: RNA
   Features Location/Qualifiers:
      - source, 1..22
         > mol_type, other RNA
         > organism, Homo sapiens
   Residues:
      caacagcaga tatgtctatg aa 22
   Sequence Number (ID): 4
   Length: 118
   Molecule Type: RNA
   Features Location/Qualifiers:
      - source, 1..118
         > mol_type, other RNA
         > organism, synthetic construct
   Residues:
      cgtggctaca gagtttcctt agcagagctg tttatagata tcgacattgc cgtgtctaaa 60 ctatcagcaa tgtcgctatc tatgcacagc tactgctagg caatccttcc ctcgataa 118
   Sequence Number (ID): 5
   Length: 22
   Molecule Type: RNA
   Features Location/Qualifiers:
      - source, 1..22
         > mol_type, other RNA
         > organism, synthetic construct
   Residues:
      tttatagata tcgacattgc cg 22
   Sequence Number (ID): 6
   Length: 22
   Molecule Type: RNA
   Features Location/Qualifiers:
      - source, 1..22
         > mol_type, other RNA
         > organism, Homo sapiens
   Residues:
      cggcaatgtc gatatctata aa 22
   Sequence Number (ID): 7
   Length: 118
   Molecule Type: RNA
   Features Location/Qualifiers:
      - source, 1..118
         > mol_type, other RNA
         > organism, synthetic construct
   Residues:
      cgtggctaca gagtttcctt agcagagctg tactattcct tggctagagt aatgtctaaa 60 ctatcaactc tagcctagga ataacacagc tactgctagg caatccttcc ctcgataa 118
   Sequence Number (ID): 8
   Length: 22
   Molecule Type: RNA
   Features Location/Qualifiers:
      - source, 1..22
         > mol_type, other RNA
         > organism, synthetic construct
   Residues:
      tactattcct tggctagagt aa 22
   Sequence Number (ID): 9
   Length: 22
   Molecule Type: RNA
   Features Location/Qualifiers:
      - source, 1..22
         > mol_type, other RNA
         > organism, Homo sapiens
   Residues:
      ttactctagc caaggaatag ta 22
   Sequence Number (ID): 10
   Length: 121
   Molecule Type: RNA
   Features Location/Qualifiers:
      - source, 1..121
         > mol_type, other RNA
         > organism, synthetic construct
   Residues:
      tgctgaaggc ttgctgtagg ctgtatgctg tttatagata tcgacattgc cgtttttgcc 60 tccaactgac ggcaagtcat atctataaac agtgtatgat gcctgttact agcattcaca 120 t 121
   Sequence Number (ID): 11
   Length: 121
   Molecule Type: RNA
   Features Location/Qualifiers:
      - source, 1..121
         > mol_type, other RNA
         > organism, synthetic construct
   Residues:
      tgctgaaggc ttgctgtagg ctgtatgctg tctcaaaatc cccgcaaatg cctttttgcc 60 tccaactgag gcattgcggg attttgagac agtgtatgat gcctgttact agcattcaca 120 t 121
   Sequence Number (ID): 12
   Length: 22
   Molecule Type: RNA
   Features Location/Qualifiers:
      - source, 1..22
         > mol_type, other RNA
         > organism, synthetic construct
   Residues:
      tctcaaaatc cccgcaaatg cc 22
   Sequence Number (ID): 13
   Length: 22
   Molecule Type: RNA
   Features Location/Qualifiers:
      - source, 1..22
         > mol_type, other RNA
         > organism, Homo sapiens
   Residues:
      ggcatttgcg gggattttga ga 22
   Sequence Number (ID): 14
   Length: 121
   Molecule Type: RNA
   Features Location/Qualifiers:
      - source, 1..121
         > mol_type, other RNA
         > organism, synthetic construct
   Residues:
      gccgctggac ccctccactc tgctgtggcc ttcagtttca taaagctggt ggattgctgt 60 
cccaaactcc accagctatt gaaactgaag gctacagtga ggggcgagct ccttctcctg 120
c 121
   Sequence Number (ID): 15
   Length: 22
   Molecule Type: RNA
   Features Location/Qualifiers:
      - source, 1..22
         > mol_type, other RNA
         > organism, synthetic construct
   Residues:
      ttcagtttca taaagctggt gg 22
   Sequence Number (ID): 16
   Length: 22
   Molecule Type: RNA
   Features Location/Qualifiers:
      - source, 1..22
         > mol_type, other RNA
         > organism, Homo sapiens
   Residues:
      ccaccagctt tatgaaactg aa 22
   Sequence Number (ID): 17
   Length: 121
   Molecule Type: RNA
   Features Location/Qualifiers:
      - source, 1..121
         > mol_type, other RNA
         > organism, synthetic construct
   Residues:
      gccgctggac ccctccactc tgctgtggcc tttatagata tcgacattgc cgattgctgt 60 
cccaaactcg gcaatgtgtt atctataaag gctacagtga ggggcgagct ccttctcctg 120
c 121
   Sequence Number (ID): 18
   Length: 121
   Molecule Type: RNA
   Features Location/Qualifiers:
      - source, 1..121
         > mol_type, other RNA
         > organism, synthetic construct
   Residues:
      gccgctggac ccctccactc tgctgtggcc tccaatagct ggtggtgacc ctattgctgt 60 
cccaaactcg ggtcacctaa gctattggag gctacagtga ggggcgagct ccttctcctg 120
c 121
   Sequence Number (ID): 19
   Length: 22
   Molecule Type: RNA
   Features Location/Qualifiers:
      - source, 1..22
         > mol_type, other RNA
         > organism, synthetic construct
   Residues:
      tccaatagct ggtggtgacc ct 22
   Sequence Number (ID): 20
   Length: 22
   Molecule Type: RNA
   Features Location/Qualifiers:
      - source, 1..22
         > mol_type, other RNA
         > organism, Homo sapiens
   Residues:
      agggtcacca ccagctattg ga 22
   Sequence Number (ID): 21
   Length: 14
   Molecule Type: RNA
   Features Location/Qualifiers:
      - source, 1..14
         > mol_type, other RNA
         > organism, Homo sapiens
   Residues:
      tgtctaaact atca 14
   Sequence Number (ID): 22
   Length: 17
   Molecule Type: RNA
   Features Location/Qualifiers:
      - source, 1..17
         > mol_type, other RNA
         > organism, Homo sapiens
   Residues:
      tttttgcctc caactga 17
   Sequence Number (ID): 23
   Length: 17
   Molecule Type: RNA
   Features Location/Qualifiers:
      - source, 1..17
         > mol_type, other RNA
         > organism, Homo sapiens
   Residues:
      attgctgtcc caaactc 17
   Sequence Number (ID): 24
   Length: 22
   Molecule Type: RNA
   Features Location/Qualifiers:
      - source, 1..22
         > mol_type, other RNA
         > organism, synthetic construct
   Residues:
      acagcagatc tgtctattta ca 22
   Sequence Number (ID): 25
   Length: 22
   Molecule Type: RNA Features Location/Qualifiers:
      - source, 1..22
         > mol_type, other RNA
         > organism, synthetic construct
   Residues:
      gcaatgtcgc tatctatgca ca 22
   Sequence Number (ID): 26
   Length: 22
   Molecule Type: RNA
   Features Location/Qualifiers:
      - source, 1..22
         > mol_type, other RNA
         > organism, synthetic construct
   Residues:
      actctagcct aggaataaca ca 22
   Sequence Number (ID): 27
   Length: 22
   Molecule Type: RNA
   Features Location/Qualifiers:
      - source, 1..22
         > mol_type, other RNA
         > organism, synthetic construct
   Residues:
      ggcattgcgg gattttgaga ca 22
   Sequence Number (ID): 28
   Length: 22
   Molecule Type: RNA
   Features Location/Qualifiers:
      - source, 1..22
         > mol_type, other RNA
         > organism, synthetic construct
   Residues:
      caccagctat tgaaactgaa gg 22
   Sequence Number (ID): 29
   Length: 22
   Molecule Type: RNA
   Features Location/Qualifiers:
      - source, 1..22
         > mol_type, other RNA
         > organism, synthetic construct
   Residues:
      gggtcaccta agctattgga gg 22
Sequence Number (ID): 30
   Length: 22
   Molecule Type: RNA
   Features Location/Qualifiers:
      - source, 1..22
         > mol_type, other RNA
         > organism, synthetic construct
   Residues:
      cggcaaguca uaucuauaaa ca 22
Sequence Number (ID): 31
   Length: 22
   Molecule Type: RNA
   Features Location/Qualifiers:
      - source, 1..22
         > mol_type, other RNA
         > organism, synthetic construct
   Residues:
      ggcaaugugu uaucuauaaa gg 22
END

## Claims

1. A nucleic acid molecule for silencing the expression of the gene encoding transmembrane serine protease 2 (TMPRSS-2) in a cell, forming a hairpin structure, comprising in the 5' to 3' direction:
- a 5' flanking sequence,
- a guide strand,
- a terminal loop,
- a passenger strand,
- a 3' flanking sequence,
wherein the terminal loop connects the guide strand and the passenger strand, which strands form a duplex of the guide strand and the passenger strand located between the terminal loop and the 5' and 3' flanking sequences, **characterized in that** said duplex comprises said guide strand having a nucleotide sequence selected from the sequences with ID numbers: 2, 5, 8, 12, 15, and 19, or a nucleotide sequence having at least 90% identity to a nucleotide sequence selected from the nucleotide sequences with ID numbers: 2, 5, 8, 12, 15, and 19, which guide strand is complementary to the mRNA encoding the TMPRSS-2 protein,
wherein said passenger strand is at least 60% complementary to said guide strand,
wherein the duplex of the guide strand and the passenger strand is located between the terminal loop of the human pri-miRNA and the 5' and 3' flanking sequences of the human pri-miRNA,
and wherein the nucleic acid molecule, upon introduction into a cell expressing the gene encoding the TMPRSS-2 protein, silences the expression of said gene.

2. A nucleic acid molecule according to claim 1, **characterized in that** the duplex comprises a short interfering RNA (siRNA) consisting of a guide strand and a passenger strand, each of which has a length of 20 to 24 nucleotides.

3. A nucleic acid molecule according to claim 1 or 2, **characterized in that** the duplex comprises a short interfering RNA (siRNA) consisting of a guide strand and a passenger strand, each of which has a length of 22 nucleotides.

4. A nucleic acid molecule according to claim 1, **characterized in that** the passenger strand has a nucleotide sequence selected from the sequences with ID numbers: 24 to 31.

5. A nucleic acid molecule according to claim 4, **characterized in that** the passenger strand is at least 77% complementary to the guide strand.

6. A nucleic acid molecule according to claim 1, **characterized in that** the guide strand is fully complementary to an mRNA encoding the TMPRSS-2 protein having a sequence selected from the sequences with ID numbers: 3, 6, 9, 13, 16, and 20, or is at least 90% complementary to mRNA encoding the TMPRSS-2 protein having a sequence selected from the sequences with ID numbers: 3, 6, 9, 13, 16, and 20.

7. A nucleic acid molecule according to any of claims 1 to 6, **characterized in that** it comprises a terminal loop, a 5' flanking sequence, and a 3' flanking sequence of a human pri-miRNA selected from pri-miRNA-122, pri-miRNA-155, pri-miRNA-135b, pri-miRNA-21, and pri-miRNA-30a, preferably a pri-miRNA selected from pri-miRNA-122 or pri-miRNA-155, wherein the terminal loop, the 5' flanking sequence, and the 3' flanking sequence of the human pri-miRNA are truncated or are not truncated.

8. A nucleic acid molecule according to claim 7, **characterized in that** the terminal loop comprises from 4 to 20 nucleotides, preferably the terminal loop comprises from 14 to 17 nucleotides.

9. A nucleic acid molecule according to claim 7 or 8, **characterized in that** the terminal loop has a sequence selected from the sequences having ID Nos. 21-23.

10. A nucleic acid molecule according to any of claims 1 to 9, **characterized in that** it constitutes an artificial microRNA (amiRNA).

11. An expression cassette for silencing the expression of the gene encoding the TMPRSS-2 protein, comprising a regulated or constitutive promoter, **characterized in that** said promoter is functionally linked to a nucleic acid sequence as defined in any of claims 1 to 10.

12. An expression cassette according to claim 11, **characterized in that** the promoter is RNA polymerase II (RNA pol II) or RNA polymerase III (RNA pol III), wherein preferably the RNA pol II promoter is a promoter selected from the cytomegalovirus (CMV) promoter or the CAG promoter consisting of a CMV enhancer and a chicken beta-actin promoter.

13. An expression cassette according to claim 11 or 12, **characterized in that** it further comprises a polyadenylation signal selected from the human growth hormone polyadenylation signal (hGH poly A), the late polyadenylation signal of simian virus 40 (SV40L poly A), or short poly A; and a post-transcriptional regulatory element of the marmot hepatitis virus (WPRE).

14. An expression vector for silencing the expression of the gene encoding the TMPRSS-2 protein, **characterized in that** it comprises a nucleic acid molecule as defined in any of claims 1 through 10 or an expression cassette as defined in any of claims 11 through 13.

15. An expression vector according to claim 14, **characterized in that** it is a vector selected from the group consisting of an adeno-associated virus (AAV), a lentivirus, an adenovirus, a plasmid, an anellavirus, a non-viral DNA vector, a minimal DNA vector, and a non-integrating lentivirus.

16. A pharmaceutical composition for silencing the expression of the gene encoding the TMPRSS-2 protein in an organism, **characterized in that** it comprises at least one nucleic acid molecule as defined in any of claims 1 to 10, or an expression cassette as defined in any of claims 11 to 13, or a vector as defined in claim 14 or 15, and a pharmaceutically acceptable carrier.

17. A pharmaceutical composition according to claim 16, **characterized in that** it comprises a nucleic acid molecule comprising a duplex of a guide strand and a passenger strand, wherein the guide strand has a nucleotide sequence selected from the sequences having ID Nos. 2, 5, 8, 12, 15, and 19, or a nucleotide sequence having at least 90% identity to the nucleotide sequence selected from the nucleotide sequences with ID numbers: 2, 5, 8, 12, 15, and 19, which guide strand is complementary to the mRNA encoding the TMPRSS-2 protein, wherein said passenger strand is at least 60% complementary to said guide strand, preferably said passenger strand is 77% complementary to said guide strand, and wherein the duplex of the guide strand and the passenger strand is located between the terminal loop of the human pri-miRNA and the 5' and 3' flanking sequences of the human pri-miRNA, wherein, preferably, the human pri-miRNA is selected from pri-miRNA-122, pri-miRNA-155, pri-miRNA-135b, pri-miRNA-21, pri-miRNA-30a, and more preferably the human pri-miRNA is selected from pri-miRNA-122 or pri-miRNA-155.

18. A pharmaceutical composition according to claim 16 or 17, **characterized in that** it comprises a nucleic acid molecule comprising a duplex of a guide strand and a passenger strand, wherein the guide strand is fully complementary to an mRNA encoding the TMPRSS-2 protein having a sequence selected from the sequences with ID numbers: 3, 6, 9, 13, 16, and 20, or is at least 90% complementary to mRNA encoding the TMPRSS-2 protein having a sequence selected from the sequences with ID numbers: 3, 6, 9, 13, 16, and 20.

19. A pharmaceutical composition according to any of claims 16 to 18, **characterized in that** it comprises a nucleic acid molecule containing a passenger strand, wherein the passenger strand has a nucleotide sequence selected from the sequences having ID numbers: 24 to 31.

20. A pharmaceutical composition according to any of claims 16 through 19, **characterized in that** the pharmaceutically acceptable carrier is selected from a stabilizing buffer, a protective protein, a stability-enhancing agent, and an agent for *in vivo* administration; **wherein the stabilizing buffer is selected from phosphate-buffered saline** (PBS), Tris-HCl, HEPES, NaCl, MgCl₂, KCl, **and** protective sugars such as sucrose and trehalose; **wherein** the protective protein is human serum albumin (HSA); **wherein** the stability-enhancing agent is selected from Polysorbate and polyethylene glycol (PEG), preferably Polysorbate 80; **wherein** the *in vivo* delivery agent is selected from lipids, nanoparticles, hydrogel, and microspheres.

21. A nucleic acid molecule as defined in any of claims 1 to 10 for use in therapy.

22. A nucleic acid molecule as defined in any of claims 1 to 10 for use in the treatment and/or prevention of a viral disease dependent on transmembrane serine protease 2 (TMPRSS-2).

23. A nucleic acid molecule for use according to claim 22, **characterized in that** the viral disease dependent on transmembrane serine protease 2 (TMPRSS-2) is a disease selected from severe acute respiratory syndrome (SARS), Middle East Respiratory Syndrome (MERS), Coronavirus Disease 2019 (COVID-19), influenza, parainfluenza, respiratory syncytial virus (RSV) infection, and Zika fever.

24. A method for *in vitro* silencing of the gene encoding the transmembrane serine protease 2 (TMPRSS-2) protein in a cell, **characterized in that** it comprises the steps in which:
(a) a nucleic acid molecule as defined in any of claims 1 through 10, or an expression cassette as defined in any of claims 11 through 13, or a vector as defined in claim 14 or 15, is introduced into a cell, and
(b) the cell containing the introduced nucleic acid molecule or expression cassette or vector from step (a) is maintained under conditions and for a time sufficient to achieve silencing of the expression of the gene encoding the TMPRSS-2 protein in the cell.

25. An *in vitro* method for silencing expression according to Claim 24, **characterized in that,** in step a), the nucleic acid molecule comprises a duplex of a guide strand and a passenger strand, wherein the guide strand has a nucleotide sequence selected from the sequences with ID numbers: 2, 5, 8, 12, 15, and 19, or a nucleotide sequence having at least 90% identity to a nucleotide sequence selected from the nucleotide sequences with ID numbers: 2, 5, 8, 12, 15, and 19, wherein said guide strand is complementary to the mRNA encoding the TMPRSS-2 protein, and wherein said passenger strand is at least 60% complementary to said guide strand, preferably said passenger strand is 77% complementary to said guide strand, and wherein the duplex of the guide strand and the passenger strand is located between the terminal loop of the human pri-miRNA and the 5' and 3' flanking sequences of the human pri-miRNA, wherein, preferably, the human pri-miRNA is selected from pri-miRNA-122, pri-miRNA-155, pri-miRNA-135b, pri-miRNA-21, pri-miRNA-30a, and more preferably the human pri-miRNA is selected from pri-miRNA-122 or pri-miRNA-155.

26. An *in vitro* method for silencing expression according to claim 24 or 25, **characterized in that** in step a) the nucleic acid molecule comprises a duplex of a guide strand and a passenger strand, wherein the guide strand is fully complementary to mRNA encoding the TMPRSS-2 protein having a sequence selected from the sequences with ID Nos.: 3, 6, 9, 13, 16, and 20, or is at least 90% complementary to mRNA encoding the TMPRSS-2 protein having a sequence selected from the sequences with ID numbers: 3, 6, 9, 13, 16, and 20.

27. An *in vitro* method for silencing expression according to any of claims 24 to 26, **characterized in that** in step a), the nucleic acid molecule contains a passenger strand, wherein the passenger strand has a nucleotide sequence selected from the sequences having ID numbers: 24 to 31.

28. An *in vitro* method for silencing expression according to any of claims 24 to 27, **characterized in that** silencing of the expression of the gene encoding the TMPRSS-2 protein is achieved using an RNA interference (RNAi) method based on artificial microRNA (amiRNA).
